(19) Europäisches Patentamt
European Patent Office
Office européen des brevets

(11) Veröffentlichungsnummer: **0 209 854**
**A2**

(12) # EUROPÄISCHE PATENTANMELDUNG

(21) Anmeldenummer: 86109848.1

(22) Anmeldetag: 17.07.86

(51) Int. Cl.⁴: C 07 D 213/30
C 07 D 213/50, C 07 D 213/48
C 07 D 213/42, C 07 D 213/89
C 07 D 405/04, C 07 D 405/06
A 01 N 43/40

(30) Priorität: 22.07.85 CH 3177/85
22.05.86 CH 2065/86

(43) Veröffentlichungstag der Anmeldung:
28.01.87 Patentblatt 87/5

(84) Benannte Vertragsstaaten:
AT BE CH DE FR GB IT LI NL

(71) Anmelder: F. HOFFMANN-LA ROCHE & CO.
Aktiengesellschaft

CH-4002 Basel(CH)

(72) Erfinder: Zehnder, Beat, Dr.
Brunngasse 89
CH-4153 Reinach(CH)

(74) Vertreter: Lederer, Franz, Dr. et al,
Vanderwerth, Lederer & Riederer Patentanwälte
Lucile-Grahn-Strasse 22
D-8000 München 80(DE)

(54) **Pyridinderivate und deren Verwendung als fungizide Wirkstoffe.**

(57) Die Erfindung betrifft neue Pyridinderivate der Formel

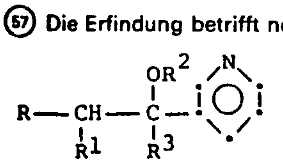

I

worin R, $R^1$, $R^2$ und $R^3$ die in der Beschreibung angegebenen Bedeutungen besitzen, deren N-Amino-Salze und Säureadditionssalze, Verfahren zu deren Herstellung, fungizide Mittel, die diese Verbindungen als Wirkstoffe enthalten, sowie die Verwendung solcher Verbindungen bzw. Mittel zur Bekämpfung von Fungi in der Landwirtschaft und im Gartenbau. Die Erfindung betrifft ebenfalls gewisse zur Herstellung der Verbindungen I dienende Ausgangsmaterialien, die zum Teil fungizide Eigenschaften aufweisen, sowie solche Wirkstoffe enthaltende fungizide Mittel.

EP 0 209 854 A2

Croydon Printing Company Ltd.

0209854

F. HOFFMANN-LA ROCHE & CO. Aktiengesellschaft, Basel/Schweiz

17. Juli 1986

RAN 6103/41

# Pyridinderivate und deren Verwendung als fungizide Wirkstoffe

Die vorliegende Erfindung betrifft heterocyclische Verbindungen, und zwar Pyridinderivate der allgemeinen Formel

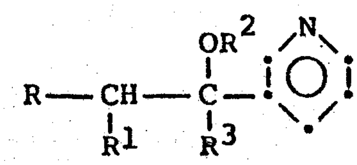

I

worin

R mono-, di- oder trisubstituiertes Phenyl, wobei die Substituenten 1-3 Halogenatome und/oder 1 oder 2 $C_{1-3}$-Alkylgruppen und/oder 1 oder 2 $C_{1-3}$-Alkoxygruppen und/oder 1 oder 2 Trifluormethylgruppen sind,

$R^1$ Wasserstoff, $C_{1-6}$-Alkyl, $C_{2-6}$-Alkenyl oder $C_{2-6}$-Alkinyl,

$R^2$ Wasserstoff,

$R^3$ eine der Gruppen (a) bis (e)

$-CO-R^4$ (a), $-C(OR^5)=CHR^6$ (b),

$-CH(R^4)OR^5$ (c), $-C(R^4)=NOR^7$ (d),

Pa/5.5.86

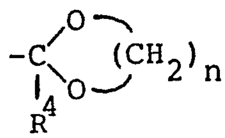 (e)

$R^4$ Wasserstoff oder $C_{1-5}$-Alkyl,
$R^5$ $C_{1-4}$-Alkyl,
$R^6$ und $R^7$ Wasserstoff oder $C_{1-4}$-Alkyl, und
n 2 oder 3 bedeuten, oder
$R^2$ zusammen mit $R^4$ der Gruppe (a), (c), (d) oder (e) Vinylen (-CH=CH-) bedeutet,

sowie die N-Amino-Salze und Säureadditionssalze der Verbindungen der Formel I.

Die Verbindungen der Formel I und deren N-Amino-Salze und Säureadditionssalze besitzen fungizide Eigenschaften und eignen sich als fungizide Wirkstoffe, insbesondere zur Verwendung in der Landwirtschaft und im Gartenbau.

Die Erfindung betrifft ferner ein Verfahren zur Herstellung der Verbindungen der Formel I und von deren N-Amino-Salzen und Säureadditionssalzen, d.h. der erfindungsgemässen Verbindungen, fungizide Mittel, die solche Verbindungen als Wirkstoffe enthalten, sowie die Verwendung solcher Verbindungen und Mittel zur Bekämpfung von Fungi in der Landwirtschaft und im Gartenbau.

In der obigen Formel I umfasst der Ausdruck "Halogen" Fluor, Chlor, Brom und Jod. Die Alkyl-, Alkenyl- und Alkinylgruppen können geradkettig oder verzweigt sein, wobei dies auch für den Alkylteil der Alkoxygruppe gilt. In di- oder trisubstituiertem Phenyl können die Substituenten gleich oder verschieden sein.

Unter dem Ausdruck "N-Amino-Salze" sind die Verbindungen der Formel I zu verstehen, deren 3-Pyridylgruppe gemäss der nachfolgenden Formel modifiziert ist:

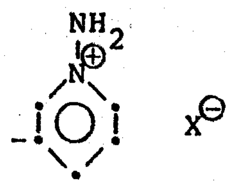

worin $X^{\ominus}$ das Anion einer physiologisch verträglichen Säure bedeutet.

Beispiele solcher Säuren sind Alkancarbonsäuren, Benzoesäure und deren kernsubstituierte Derivate, wie beispielsweise alkyl-, nitro- und/oder chlorsubstituierte Benzoesäuren, gegebenenfalls substituierte Benzolsulfonsäure, Carbaminsäure, Salzsäure, Bromwasserstoffsäure, Jodwasserstoffsäure und Schwefelsäure.

Als Säureadditionssalze der Verbindungen der Formel I kommen physiologisch verträgliche Salze in Frage. Hierzu gehören vorzugsweise Salze dieser Verbindungen mit anorganischen und organischen Säuren, wie Salzsäure; Salpetersäure; Phosphorsäure; mono- und bifunktionellen Carbonsäuren und Hydroxycarbonsäuren, z.B. Essigsäure, Maleinsäure, Bernsteinsäure, Fumarsäure, Weinsäure, Zitronensäure, Salicylsäure, Sorbinsäure und Milchsäure; und Sulfonsäuren, z.B. 1,5-Naphthalin-disulfonsäure.

Das Vorhandensein mindestens eines asymmetrischen Kohlenstoffatoms in den erfindungsgemässen Verbindungen hat zur Folge, dass die Verbindungen als optische Antipoden auftreten können. Durch das Vorliegen einer allfälligen aliphatischen C=C- oder C=N-Doppelbindung kann auch geometrische Isomerie auftreten. Die Formel I soll all diese möglichen isomeren Formen und deren Gemische umfassen.

Unabhängig voneinander bedeuten R vorzugsweise Mono-, Di- oder Trihalogenphenyl, insbesondere 2,4-Dichlorphenyl, $R^1$ vorzugsweise Wasserstoff oder $C_{1-6}$-Alkyl, $R^2$ vorzugsweise Wasserstoff und $R^3$ vorzugsweise eine Gruppe (a) oder (b), insbesondere Acetyl bzw. 1-Methoxy- oder

1-Aethoxyvinyl. Gegenüber den N-Amino-Salzen der Verbindungen der Formel I sind die freien Pyridinderivate und deren Säureadditionssalze bevorzugt. Eine interessante Untergruppe von erfindungsgemässen Verbindungen sind diejenigen, bei denen $R^1$ der Formel I Wasserstoff bedeutet. Eine weitere solche Untergruppe besteht aus denjenigen erfindungsgemässen Verbindungen, bei denen $R^1$ der Formel I Methyl bedeutet.

Besonders bevorzugte einzelne Verbindungen der Formel I sind:

5-(2,4-Dichlorphenyl)-4-hydroxy-4-(3-pyridyl)-3-pentanon,
2-(2,4-Dichlor-α-methylbenzyl)-2-(3-pyridyl)-3(2H)-furanon,
2-(2,4-Dichlorbenzyl)-2-(3-pyridyl)-3(2H)-furanon,
4-(2,4-Dichlorphenyl)-3-hydroxy-3-(3-pyridyl)-2-pentanon,
4-(2,4-Dichlorphenyl)-3-hydroxy-3-(3-pyridyl)-2-butanon,
4-(p-Chlorphenyl)-3-hydroxy-3-(3-pyridyl)-2-pentanon,
α-(2,4-Dichlorbenzyl)-α-(1-methoxyvinyl)-3-pyridinmethanol,
α-Aethoxymethyl-α-(2,4-dichlor-α-methylbenzyl)-3-pyridinmethanol,
α-(2,4-Dichlor-α-methylbenzyl)-α-methoxymethyl-3-pyridinmethanol,
α-(2,4-Dichlorbenzyl)-α-(1-methoxyäthyl)-3-pyridinmethanol,
α-(2,4-Dichlorbenzyl)-3-pyridinglykolaldehyd-O-methyloxim und
α-(2,4-Dichlor-α-methylbenzyl)-α-[(methoxyimino)-methyl]-3-pyridinmethanol.

Weitere Vertreter von Verbindungen der Formel I sind:

3-(2,4-Dichlorphenyl)-2-hydroxy-2-(3-pyridyl)-1-butanon-äthylenketal,

α-(1-Aethoxyäthyl)-α-(2,4-dichlorbenzyl)-3-pyridinmethanol,

α-(2,4-Dichlor-α-methylbenzyl)-α-(1-methoxy-äthyl)-3-pyridinmethanol,

α-(1-Aethoxyäthyl)-α-(2,4-dichlor-α-methylbenzyl)-3-pyridinmethanol,

α-(2,4-Dichlorbenzyl)-α-(1-methoxy-2-methylpropyl)-3-pyridinmethanol,

α-(2,4-Dichlorbenzyl)-α-methoxymethyl-3-pyridinmethanol und

α-Aethoxymethyl-α-(2,4-dichlorbenzyl)-3-pyridinmethanol.

Das erfindungsgemässe Verfahren zur Herstellung der Verbindungen der Formel I und von ihren N-Amino-Salzen und Säureadditionssalzen ist dadurch gekennzeichnet, dass man

a) zwecks Herstellung derjenigen Verbindungen der Formel I, in denen $R^2$ Wasserstoff, $R^3$ eine Gruppe (a) und $R^4$ Wasserstoff oder $C_{1-5}$-Alkyl bedeuten, ein Dithianderivat der allgemeinen Formel

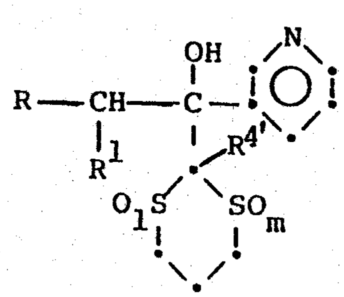

II

worin

R und $R^1$ die oben angegebenen Bedeutung besitzen,

$R^{4'}$ Wasserstoff oder $C_{1-5}$-Alkyl bedeutet und

l und m unabhängig voneinander 0 oder 1 bedeuten,

einer Hydrolyse in saurem Milieu unterwirft.

b) zwecks Herstellung derjenigen Verbindungen der Formel I, in denen $R^3$ eine Gruppe (a) und $R^4$ $C_{1-5}$-Alkyl oder zusammen mit $R^2$ Vinylen bedeuten, ein Alkin der allgemeinen Formel

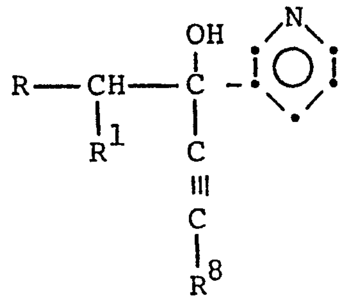

III

worin

| | |
|---|---|
| R und $R^1$ | die oben angegebenen Bedeutungen besitzen, |
| $R^8$ | Wasserstoff, $C_{1-4}$-Alkyl oder eine Gruppe |

$$-CH(OR^9)_2$$

bedeutet und

$R^9$ einen niederen Alkylrest, insbesondere $C_{1-4}$-Alkyl, bedeutet,

einer Hydrolyse in saurem Milieu unterwirft,

c) zwecks Herstellung derjenigen Verbindungen der Formel I, in denen $R^3$ eine Gruppe (b) bedeutet, ein Keton der allgemeinen Formel

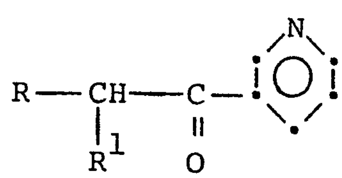

IV

worin R und $R^1$ die oben angegebenen Bedeutungen besitzen,

mit einem geeigneten Metallsalz, insbesondere dem Lithiumsalz, eines Enoläthers der allgemeinen Formel

$$CH(OR^5)=CHR^6 \qquad V$$

worin $R^5$ und $R^6$ die oben angegebenen Bedeutungen besitzen,

umsetzt,

d) zwecks Herstellung derjenigen Verbindungen der allgemeinen Formel I, in denen $R^2$ Wasserstoff, $R^3$ eine Gruppe (a) und $R^4$ $C_{1-5}$-Alkyl bedeuten, einen Enoläther der allgemeinen Formel

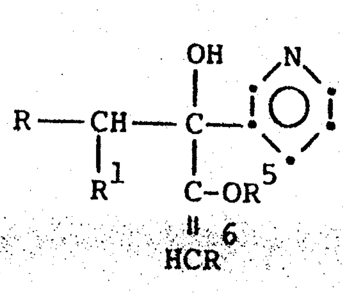

I'

worin

R, $R^1$, $R^5$ und $R^6$ die oben angegebenen Bedeutungen besitzen,

einer Hydrolyse in saurem Milieu unterwirft,

e) zwecks Herstellung derjenigen Verbindungen der Formel I, in denen $R^3$ eine Gruppe (c) bedeutet, einen Alkohol der allgemeinen Formel

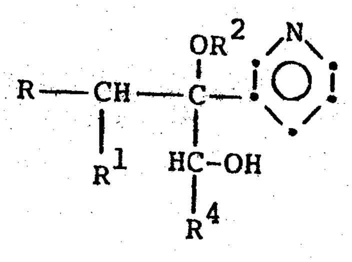

VI

worin

R, $R^1$, $R^2$ und $R^4$ die oben angegebenen Bedeutungen besitzen,

mit einem Alkylierungsmittel behandelt,

f) zwecks Herstellung derjenigen Verbindungen der Formel I, in denen $R^2$ Wasserstoff, $R^3$ eine Gruppe (c) und $R^4$ $C_{1-5}$-Alkyl bedeuten, einen Enoläther der oben angegebenen

allgemeinen Formel I' katalytisch hydriert,

g) zwecks Herstellung derjenigen Verbindungen der Formel I, in denen $R^2$ Wasserstoff, $R^3$ eine Gruppe (c) und $R^4$ $C_{1-5}$-Alkyl bedeuten, ein Keton der oben angegebenen allgemeinen Formel IV mit einem geeigneten Metallsalz, insbesondere dem Lithiumsalz, eines Dialkyläthers der allgemeinen Formel

$$R^{4''}CH_2OR^5 \qquad VII$$

worin

$R^{4''}$ $C_{1-5}$-Alkyl bedeutet und

$R^5$ die oben angegebene Bedeutung besitzt,

umsetzt,

h) zwecks Herstellung derjenigen Verbindungen der Formel I, in denen $R^3$ eine Gruppe (d) bedeutet, einen Aldehyd bzw. ein Keton der allgemeinen Formel

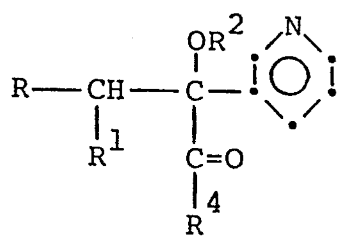

I"

worin R, $R^1$, $R^2$ und $R^4$ die oben angegebenen Bedeutungen besitzen,

mit einem Amin der allgemeinen Formel

$$H_2N\text{-}OR^7 \qquad VIII$$

worin $R^7$ die oben angegebene Bedeutung besitzt,

oder mit einem Salz dieses Amins mit einer starken Säure

umsetzt,

i) zwecks Herstellung derjenigen Verbindungen der Formel I, in denen $R^3$ eine Gruppe (e) bedeutet, einen Aldehyd bzw. ein Keton der oben angegebenen allgemeinen Formel I" mit Aethylenglykol bzw. Propandiol-1,3 umsetzt

und zwecks Herstellung eines N-Amino-Salzes einer Verbindung der Formel I diese mit einem Aminderivat der allgemeinen Formel

$$H_2N-X \qquad IX$$

wobei $X^{\ominus}$ das Anion einer physiologisch verträglichen Säure bedeutet,

N-aminiert

und zwecks Herstellung eines Säureadditionssalzes einer Verbindung der Formel I die Verbindung der Formel I mit einer Säure umsetzt.

Die Verfahrensvariante a) wird zweckmässigerweise durchgeführt, indem man das Dithianderivat der Formel II in verdünnter wässriger Mineralsäure, vorzugsweise 2-20%iger Schwefelsäure, bei Temperaturen zwischen Raumtemperatur und der Rückflusstemperatur des Reaktionsgemisches hydrolysiert. In vielen Fällen erweist es sich als vorteilhaft, die Hydrolyse zusätzlich in Gegenwart einer katalytischen Menge eines Schwermetallsalzes, z.B. von Quecksilbersulfat, durchzuführen. Im Falle der Hydrolyse eines Dithianderivats der Formel II, in der $R^1$ $C_{2-6}$-Alkinyl bedeutet, erfolgt die Hydrolyse unter milden Reaktionsbedingungen, weil von einem "oxidierten" Dithianderivat ausgegangen wird, d.h. l und m der Formel II bedeuten 1.

Die Verfahrensvariante b) erfolgt zweckmässigerweise unter den oben im Zusammenhang mit der Verfahrensvariante a)

beschriebenen Reaktionsbedingungen, allerdings sind im Falle eines Alkins der Formel III, in der $R^8$ $-CH(OR^9)_2$ bedeutet, relativ milde Reaktionsbedingungen angezeigt, und ein Zusatz eines Schwermetallsalzes ist zu vermeiden.

Die Enolätheraddition nach Verfahrensvariante c) wird zweckmässigerweise so durchgeführt, dass man das Keton der Formel IV zum Metallsalz, insbesondere zum Lithiumsalz, des Enoläthers der Formel V in einem inerten Verdünnungsmittel, vorzugsweise einem aprotischen organischen Lösungsmittel, wie einem aliphatischen oder cyclischen Aether, z.B. Diäthyläther, Tetrahydrofuran oder Dioxan, in einem Temperaturbereich von ca. -80°C bis 0°C gibt und reagieren lässt. In vielen Fällen erweist es sich als vorteilhaft, das Metallsalz im Ueberschuss zu verwenden, und zwar bis zu 200 Molprozent. Zweckmässigerweise wird das Salz vor Zugabe des Ketons in situ hergestellt, beispielsweise durch langsame Zugabe von tert.-Butyllithium in einem organischen Lösungsmittel, vorzugsweise einem Kohlenwasserstoff, z.B. n-Hexan, zu einer Lösung des Enoläthers der Formel V in demselben Lösungsmittel, in welchem hierauf die Umsetzung mit dem Keton IV erfolgt, und bei niedrigen Temperaturen, also im unteren Bereich des obenerwähnten Temperaturintervalls. Danach kann zur Beschleunigung der Reaktion die Temperatur des Reaktionsgemisches langsam erhöht werden, beispielsweise bis ca. 0°C, und anschliessend das Gemisch abgekühlt und das Keton IV zugegeben werden.

Die Hydrolyse nach Verfahrensvariante d) wird zweckmässigerweise durch Behandlung des Enoläthers der Formel I' in einem polaren organischen Verdünnungsmittel, wie einem cyclischen Aether, z.B. Tetrahyrofuran oder Dioxan, einem niederen aliphatischen Amid, z.B. Dimethylformamid, oder einem niederen Alkanol, z.B. Methanol, Aethanol oder Isopropanol, mit wässriger Säure, wie Salzsäure, Schwefelsäure oder Trifluoressigsäure, bei Temperaturen zwischen 0°C und

der Rückflusstemperatur des Reaktionsgemisches durchgeführt.

Die Verätherung nach Verfahrensvariante e) wird zweckmässigerweise unter Verwendung eines $C_{1-4}$-Alkylchlorids, -bromids, -jodids, -mesylats oder -tosylats oder eines Di($C_{1-4}$-alkyl)-sulfats als Alkylierungsmittel, in einem Verdünnungsmittel und in Gegenwart einer Base durchgeführt. Das Verdünnungsmittel ist vorzugsweise ein polares aprotisches organisches Lösungsmittel, wie ein aliphatischer oder cyclischer Aether, z.B. Dimethoxyäthan oder Tetrahydrofuran, oder ein Dialkylamid, z.B. Dimethylformamid, und die Base vorzugsweise eine starke, wie Natriumhydrid, Calciumhydrid oder Lithiumdiisopropylamid. Ebenso kann der Alkohol der Formel VI in einem Zweiphasengemisch von wässrigem Alkalimetallhydroxid, wie Natronlauge, und einem Halogenkohlenwasserstoff, wie Methylenchlorid oder Chloroform, mittels eines Phasentransferkatalysators, z.B. Tetrabutylammoniumhydrogensulfat, mit dem Alkylierungsmittel umgesetzt werden. In jedem Fall erfolgt die Umsetzung zweckmässigerweise bei Temperaturen zwischen 0°C und der Rückflusstemperatur des Reaktionsgemisches.

Die Hydrierung nach Verfahrensvariante f) wird zweckmässigerweise unter Verwendung von Palladium oder Platin als Katalysator durchgeführt, wobei sich der Katalysator geeigneterweise auf einem inerten Träger, wie Calciumcarbonat oder Aktivkohle, befindet. Zudem wird zweckmässigerweise in Gegenwart eines organischen Verdünnungsmittels gearbeitet. Es kommen als solche Verdünnungsmittel insbesondere niedere Alkanole, z.B. Methanol und Aethanol; aliphatische Carbonsäuren, z.B. Essigsäure; aliphatische Ketone, z.B. Aceton und 2-Butanon; halogenierte aliphatische Kohlenwasserstoffe, z.B. Methylenchlorid und Chloroform; und Aromaten, z.B. Toluol und Xylole, in Frage. Die Hydrierung erfolgt zweckmässigerweise in einem Temperaturbereich von 0°C bis zur Rückflusstemperatur des Reaktionsgemisches und unter einem

Wasserstoffdruck von einer oder mehreren Atmosphären.

Die Addition des Enoläthers nach Verfahrensvariante g) wird zweckmässigerweise so durchgeführt, dass man das Keton der Formel IV zum Metallsalz, insbesondere zum Lithiumsalz, des Dialkyläthers der Formel VII in einem inerten Verdünnungsmittel, vorzugsweise in einem aprotischen organischen Lösungsmittel, wie einem aliphatischen oder cyclischen Aether, z.B. Tetrahydrofuran, Dioxan oder demselben Dialkyläther VII, in einem Temperaturbereich von ca. -100°C bis zur Raumtemperatur, vorzugsweise bei ca. -75°C, gibt und reagieren lässt. Es erweist sich als vorteilhaft, das Metallsalz vor Zugabe des Ketons IV in situ herzustellen, beispielsweise indem man den Dialkyäther VII mit einer starken Base, wie einer Alkyllithiumverbindung, gegebenenfalls unter Zusatz eines Alkoholats, wie Kalium-tert.butylat, und/oder eines Alkalihalogenids, wie Lithiumbromid, versetzt, und zwar bei niedrigen Temperaturen, also in unterem Bereich des obenerwähnten Temperaturintervalls. Hierauf wird das Keton IV zugegeben. In dieser Verfahrensvariante wird vorzugsweise ein symmetrischer Dialkyläther VII oder ein Dialkyläther VII, in dem $R^5$ tert.Butyl bedeutet, eingesetzt.

Die Oximbildung nach Verfahrensvariante h) erfolgt zweckmässigerweise in einem polaren organischen Verdünnungsmittel, wie einem niederen Alkanol, z.B. Methanol oder Aethanol; einem Dialkylamid, z.B. Dimethylformamid; oder einem tertiären Amin, z.B. Pyridin, bei Temperaturen zwischen 0°C und der Rückflusstemperatur des Reaktionsgemisches. Falls das Amin der Formel VIII in Form eines Säureadditionssalzes, z.B. des Hydrochlorids oder Hydrogensulfats, eingesetzt wird, wird dem Reaktionsgemisch zweckmässigerweise eine Base, wie Natrium- oder Kaliumcarbonat, Triäthylamin oder Pyridin, zugesetzt, es sei denn, das verwendete Verdünnungsmittel ist selbst basisch.

Die Ketalisierung nach Verfahrensvariante i) wird zweckmässigerweise in einem organischen Verdünnungsmittel, in Gegenwart einer Säure und in einem Temperaturbereich zwischen 80°C und 140°C durchgeführt. Als Verdünnungsmittel eignen sich insbesondere aromatische Kohlenwasserstoffe, z.B. Benzol, Toluol und Xylole. Solche Verdünnungsmittel erlauben insbesondere die kontinuierliche destillative Entfernung des gebildeten Wassers aus dem Reaktionsgemisch. Als Säuren kommen insbesondere Mineralsäuren, wie Schwefelsäure und Salzsäure, oder Sulfonsäuren, wie p-Toluolsulfonsäure, in Frage. Der Dialkohol wird vorzugsweise im Ueberschuss verwendet.

Die N-Aminierung einer Verbindung der Formel I mit einem Aminderivat der Formel IX wird zweckmässigerweise so durchgeführt, dass man die Verbindung I mit dem Aminderivat in einem inerten organischen Verdünnungsmittel, wie einem halogenierten aliphatischen Kohlenwasserstoff, z.B. Methylenchlorid oder Chloroform, bei Temperaturen zwischen 0°C und der Rückflusstemperatur des Reaktionsgemisches behandelt.

Zur Herstellung der Säureadditionssalze der Verbindungen der Formel I werden die Verbindungen I mit den gewünschten Säuren auf übliche Weise umgesetzt.

Die Isolierung und Reinigung der so hergestellten Verbindungen der Formel I und deren N-Amino-Salze und Säureadditionssalze können nach an sich bekannten Methoden erfolgen.

Die als Ausgangsmaterialien in der Verfahrensvariante a) verwendeten Dithianderivate der Formel II sind neu und können dadurch hergestellt werden, dass man ein Keton der oben angegebenen allgemeinen Formel IV mit einem Dithian--Salz der allgemeinen Formel

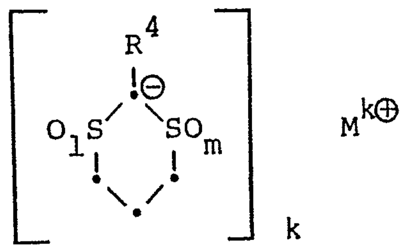 X

worin

$R^4$, 1 und m die oben angegebenen Bedeutungen besitzen,

$M^{k\oplus}$ ein Alkalimetall- oder Erdalkalimetallkation bedeutet, und

k die Wertigkeit des Kations M bedeutet,

umsetzt. Das Alkali- bzw. Erdalkalimetall ist vorzugsweise Lithium, Natrium oder Kalium bzw. Calcium oder Magnesium. Die Umsetzung erfolgt zweckmässigerweise unter Verwendung eines inerten organischen Verdünnungsmittels, insbesondere eines aprotischen organischen Lösungsmittels, wie eines aliphatischen oder cyclischen Aethers, z.B. Diäthyläther, Tetrahydrofuran oder Dioxan, in einem Temperaturbereich von ca. -80°C bis 0°C. In vielen Fällen erweist es sich als vorteilhaft, das Dithian-Salz der Formel X im Ueberschuss zu verwenden, und zwar vorzugsweise bis zu 200 Molprozent.

Die Ausgangsmaterialien der Formeln III und IV sind entweder bekannt oder können nach an sich bekannten Methoden hergestellt werden (siehe z.B. die Europäische Patentpublikation Nr. 137.456). Ebenfalls bekannt oder nach an sich bekannten Methoden herstellbar sind die Ausgangsmaterialien bzw. Zwischenprodukte der Formeln V und VII-X.

Die als Ausgangsmaterialien in der Verfahrensvariante e) dienenden Alkohole der Formel VI sind neu und können dadurch hergestellt werden, dass man einen Aldehyd bzw. ein Keton der oben angebenen allgemeinen Formel I" reduziert. Die Reduktion erfolgt vorzugsweise mittels eines komplexen Metallhydrids, wie Natriumborhydrid, wobei in einem protischen organischen Verdünnungsmittel, wie einem Alkohol, z.B. Methanol oder Aethanol, oder einer aliphatischen Carbonsäure, z.B. Essigsäure, bei Temperaturen um Raumtemperatur gearbeitet wird, oder Lithiumaluminiumhydrid, das üblicherweise in einem aprotischen organischen Verdünnungsmittel, insbesondere einem aliphatischen oder cyclischen Aether, z.B. Diäthyläther, Tetrahydrofuran oder Dioxan, bei Temperaturen zwischen 0°C und Raumtemperatur eingesetzt wird. Als komplexe Metallhydride in geeigneten Verdünnungsmitteln kommen auch Lithiumborhydrid in Aethanol oder Tetrahydrofuran, Natriumborhydrid-Aluminiumchlorid in einem Aether, z.B. Diglym, und Lithiumtri(tert.butoxy)aluminiumhydrid in Tetrahydrofuran in Frage. Der Aldehyd bzw. das Keton der Formel I" kann auch z.B. mittels Diboran in Tetrahydrofuran oder mittels an sich bekannter katalytischer Hydrierung, wobei üblicherweise als Verdünnungsmittel ein gegebenenfalls halogenierter Kohlenwasserstoff verwendet wird, reduziert werden. Die Reaktionsbedingungen für die diesbezügliche Reduktion des Aldehyds bzw. Ketons I" sind dem Fachmann im allgemeinen aus analogen Reduktionen bekannt.

Diese Alkohole der Formel VI bilden einen weiteren Gegenstand der vorliegenden Erfindung.

Bei den Ausgangsmaterialien der Formeln I' und I" handelt es sich um Untergruppen der Verbindungen der Formel I: Die Enoläther der Formel I' können gemäss der Verfahrensvariante c) aus entsprechenden Ketonen der Formel IV und Metallsalzen der Enoläther der Formel V, und die Aldehyde bzw. Ketone der Formel I" gemäss den Verfahrensvarianten a), b) und d) aus entsprechenden Dithianderivaten der Formel II, Alkinen der Formel III bzw. Enoläthern der Formel I' hergestellt werden.

Die erfindungsgemässen Verbindungen, d.h. die Verbindungen der Formel I und deren N-Amino-Salze und Säureadditionssalze, besitzen fungizide Wirkung und können dementsprechend zur Bekämpfung von Pilzen in der Landwirtschaft, im Gartenbau sowie in der Holzverarbeitung Verwendung

finden. Sie eignen sich insbesondere zur Hemmung des Wachstums oder zur Vernichtung von phytopathogenen Pilzen auf Pflanzenteilen, z.B. Blättern, Stengeln, Wurzeln, Knollen, Früchten oder Blüten, und auf Saatgut sowie im Erdboden. Ferner können mit den erfindungsgemässen Verbindungen holzabbauende und holzverfärbende Pilze bekämpft werden. Die erfindungsgemässen Verbindungen sind besonders wirksam bei der Bekämpfung von Pilzen der Klassen Deuteromycetes, Ascomycetes und Basidiomycetes, wie beispielsweise Botrytis cinerea, Erysiphe cichoracearum, Erysiphe graminis, Uncinula necator, Podsophaera leucotricha, Venturia inaequalis, Cercospora archidicola, Cercospora beticola und Mycosphaerella fijiensis sowie von Schadpilzen der Gattungen Sphaerotheca, Puccinia, Uromyces, Hemileia, Rhizoctonia, Alternaria, Cercosporella, Ceratocystis (z.B. Ceratocystis ulmi und Ceratocystis fimbriata), Verticillium, Fusarium, Helmithosporium, Sclerotinia, Penicillium, Septona, Ustilago, Tilletia, Coniophora, Gloeophyllum und Aureobasidium.

Die erfindungsgemässen Verbindungen zeichnen sich durch lokale und bzw. oder systemische Wirkung aus.

Die erfindungsgemässen Verbindungen wirken gegen phytopathogene Pilze unter Gewächshausbedingungen bereits bei Konzentrationen von 1 mg bis 500 mg Wirkstoff pro Liter Spritzbrühe. Im Freiland werden vorteilhaft Konzentrationen von 25 g bis 1500 g Wirkstoff der Formel I pro Hektar und Behandlung zur Anwendung gebracht. Zur Bekämpfung von samen- oder bodenbürtigen Pilzen im Beizverfahren werden mit Vorteil 0,05 g bis 1,5 g Wirkstoff der Formel I pro kg Samen verwendet.

Die erfindungsgemässen Verbindungen können zu verschiedenartigen Mitteln, z.B. Lösungen, Suspensionen, Emulsionen, emulgierbaren Konzentraten und pulverförmigen Präparaten, formuliert werden. Die erfindungsgemässen fungiziden Mittel sind dadurch gekennzeichnet, dass sie eine wirksame

Menge mindestens einer Verbindung der allgemeinen Formel I, wie oben definiert, oder eines N-Amino-Salzes oder Säureadditionssalzes einer solchen Verbindung sowie Formulierungshilfsstoffe enthalten. Die Mittel enthalten zweckmässigerweise zumindest einen der folgenden Formulierungshilfsstoffe:

Feste Trägerstoffe; Lösungs- bzw. Dispersionsmittel; Tenside (Netz- und Emulgiermittel); Dispergatoren (ohne Tensidwirkung); und Stabilisatoren.

Als feste Trägerstoffe kommen im wesentlichen in Frage: natürliche Mineralstoffe, wie Kaolin, Tonerden, Kieselgur, Talkum, Bentonit, Kreide, z.B. Schlämmkreide, Magnesiumcarbonat, Kalkstein, Quarz, Dolomit, Attapulgit, Montmorillonit und Diatomeenerde; synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Granulate oder Pulver vorliegen können.

Als Lösungs- bzw. Dispersionsmittel kommen im wesentlichen in Frage: Aromaten, wie Toluol, Xylole, Benzol und Alkylnaphthaline; chlorierte Aromaten und chlorierte aliphatische Kohlenwasserstoffe, wie Chlorbenzole, Chloräthylene und Methylenchlorid; aliphatische Kohlenwasserstoffe, wie Cyclohexan und Paraffine, z.B. Erdölfraktionen; Alkohole, wie Butanol und Glykol, sowie deren Aether und Ester; Ketone, wie Aceton, Methyläthylketon, Methylisobutylketon und Cyclohexanon; und stark polare Lösungs- bzw. Dispersionsmittel, wie Dimethylformamid, N-Methylpyrrolidon und Dimethylsulfoxid, wobei solche Lösungs- bzw. Dispersionsmittel vorzugsweise Flammpunkte von mindestens 30°C und Siedepunkte von mindestens 50°C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie Halogenkohlenwasserstoffe, z.B. Dichlordifluormethan. Im Falle der Benutzung von Wasser als Lösungsmittel können z.B. auch organische Lösungsmittel als Hilfslösungsmittel verwendet werden.

Die Tenside (Netz- und Emulgiermittel) können nicht-ionische Verbindungen sein, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen darstellen, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-dodecylbenzolsulfonat, und Butylnaphthalinsulfonate; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage: Lignin, Natrium- und Ammoniumsalze von Ligninsulfonsäure, Natriumsalze von Maleinsäureanhydrid-Diisobutylen-Copolymeren, Natrium- und Ammoniumsalze von sulfonierten Polykondensationsprodukten aus Naphthalin und

Formaldehyd, und Sulfitablaugen.

Als Dispergatoren, die sich insbesondere als Verdickungs- bzw. Antiabsetzmittel eignen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel, z.B. Epichlorhydrin, Phenylglycidäther und Soyaepoxide; Antioxidantien, z.B. Gallussäureester und Butylhydroxytoluol; UV-Absorber, z.B. substituierte Benzophenone, Diphenylacrylnitrilsäureester und Zimtsäureester; und Deaktivatoren, z.B. Salze der Aethylendiamintetraessigsäure und Polyglykole.

Die erfindungsgemässen fungiziden Mittel können neben den Wirkstoffen der Formel I auch andere Wirkstoffe enthalten, z.B. anderweitige fungizide Mittel, insektizide und akarizide Mittel, Bakterizide, Pflanzenwachstumsregulatoren und Düngemittel. Solche Kombinationsmittel eignen sich zur Verbreiterung des Wirkungsspektrums oder zur spezifischen Beeinflussung des Pflanzenwachstums.

Im allgemeinen enthalten die erfindungsgemässen fungiziden Mittel, je nach deren Art, zwischen 0,0001 und 95 Gewichtsprozent an erfindungsgemässer Verbindung bzw. erfindungsgemässen Verbindungen als Wirkstoff(en). Sie können in einer Form vorliegen, die sich für die Lagerung und den Transport eignet. In solchen Formen, z.B. emulgierbaren Konzentraten, ist die Wirkstoffkonzentration normalerweise im höheren Bereich des obigen Konzentrationsintervalls. Diese Formen können dann mit gleichen oder verschiedenen Formulierungshilfsstoffen bis zu Wirkstoffkonzentrationen verdünnt werden, die sich für den praktischen Gebrauch eignen, und solche Konzentrationen liegen normalerweise im niedrigeren Bereich des obigen Konzentrationsintervalls.

Emulgierbare Konzentrate enthalten im allgemeinen 5 bis 95 Gewichtsprozent, vorzugsweise 25 bis 75 Gewichtsprozent, der Verbindung bzw. Verbindungen der Formel I. Als Anwendungsformen kommen u.a. gebrauchsfertige Lösungen, Emulsionen und Suspensionen, die sich beispielsweise als Spritzbrühen eignen, in Frage. In solchen Spritzbrühen können z.B. Konzentrationen zwischen 0,0001 und 20 Gewichtsprozent vorliegen. Im Ultra-Low-Volume-Verfahren können Spritzbrühen formuliert werden, in denen die Wirkstoffkonzentration vorzugsweise von 0,5 bis 20 Gewichtsprozent beträgt, während die im Low-Volume-Verfahren und im High-Volume-Verfahren formulierten Spritzbrühen vorzugsweise eine Wirkstoffkonzentration von 0,02 bis 1,0 bzw. 0,002 bis 0,1 Gewichtsprozent aufweisen.

Die erfindungsgemässen fungiziden Mittel können dadurch hergestellt werden, dass man mindestens eine Verbindung der allgemeinen Formel I bzw. ein N-Amino-Salz oder Säureadditionssalz einer solchen Verbindung mit Formulierungshilfsstoffen vermischt.

Die Herstellung der Mittel kann in bekannter Weise durchgeführt werden, z.B. durch Vermischen der Wirkstoffes mit festen Trägerstoffen, durch Auflösen oder Suspendieren in geeigneten Lösungs- bzw. Dispersionsmitteln, eventuell unter Verwendung von Tensiden als Netz- oder Emulgiermitteln oder von Dispergatoren, durch Verdünnen bereits vorbereiteter emulgierbarer Konzentrate mit Lösungs- bzw. Dispersionsmitteln, usw.

Im Falle von pulverförmigen Mitteln kann der Wirkstoff mit einem festen Trägerstoff vermischt werden, z.B. durch Zusammenmahlen; oder man kann den festen Trägerstoff mit einer Lösung oder Suspension des Wirkstoffs imprägnieren und dann das Lösungs- bzw. Dispersionsmittel durch Abdunsten, Erhitzen oder durch Absaugen unter vermindertem Druck entfernen. Durch Zusatz von Tensiden bzw. Dispergatoren kann

man solche pulverförmige Mittel mit Wasser leicht benetzbar machen, so dass sie in wässrige Suspensionen, die sich z.B. als Spritzmittel eignen, übergeführt werden können.

Die erfindungsgemässen Verbindungen können auch mit einem Tensid und einem festen Trägerstoff zur Bildung eines netzbaren Pulvers vermischt werden, welches in Wasser dispergierbar ist, oder sie können mit einem festen vorgranulierten Trägerstoff zur Bildung eines granulatförmigen Produktes vermischt werden.

Wenn gewünscht, kann eine erfindungsgemässe Verbindung in einem mit Wasser nicht mischbaren Lösungsmittel, wie beispielsweise einem alicyclischen Keton, gelöst werden, das zweckmässigerweise gelöste Emulgiermittel enthält, so dass die Lösung bei Zugabe zu Wasser selbstemulgierend wirkt. Andernfalls kann der Wirkstoff mit einem Emulgiermittel vermischt und das Gemisch dann mit Wasser auf die gewünschte Konzentration verdünnt werden. Zudem kann der Wirkstoff in einem Lösungsmittel gelöst und danach mit einem Emulgiermittel gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die Verwendung der erfindungsgemässen Mittel kann nach den im Pflanzenschutz bzw. in der Landwirtschaft üblichen Applikationsmethoden erfolgen. Das erfindungsgemässe Verfahren zur Bekämpfung von Fungi ist dadurch gekennzeichnet, dass man das zu schützende Gut, z.B. Pflanzen, Pflanzenteile bzw. Samen, mit einer wirksamen Menge einer erfindungsgemässen Verbindung bzw. eines erfindungsgemässen Mittels behandelt.

Auch gewisse Verbindungen der Formel VI besitzen fungizide Wirkung und können analog zu den Verbindungen der Formel I und ihren N-Amino-Salzen und Säureadditionssalzen

zur Bekämpfung von Pilzen in der Landwirtschaft, im Gartenbau sowie in der Holzverarbeitung eingesetzt werden. Die fraglichen Verbindungen VI weisen also ein ähnliches Wirkungsspektrum wie die Verbindungen der Formel I, deren N-Amino-Salze und Säureadditionssalze auf, und können wie diese Verbindungen zu verschiedenartigen fungiziden Mitteln formuliert und verwendet werden.

Aufgrund ihrer besonders ausgeprägten fungiziden Aktivität sind die folgenden Verbindungen der Formel VI bevorzugt:

4-(2,4-Dichlorphenyl)-3-(3-pyridyl)-2,3-butandiol,
1-(2,4-Dichlorbenzyl)-1-(3-pyridyl)-1,2-äthandiol,
5-(2,5-Dichlorphenyl)-4-(3-pyridyl)-3,4-hexandiol,
3-(2,4-Dichlorphenyl)-2-(3-pyridyl)-1,2-butandiol,
4-(2,4-Dichlorphenyl)-3-(3-pyridyl)-6-heptin-2,3-diol,
4-(2,4-Dichlorphenyl)-3-(3-pyridyl)-2,3-pentandiol,
4-(2,4-Dichlorphenyl)-3-(3-pyridyl)-6-hepten-2,3-diol

und

4-(2,4-Dichlorphenyl)-3-(3-pyridyl)-2,3-hexandiol.

Die ersten vier genannten Verbindungen VI sind besonders bevorzugte fungizide Wirkstoffe.

Die nachstehenden Beispiele illustrieren die Erfindung.

## I. <u>Herstellung der Wirkstoffe der Formel I:</u>

### <u>Beispiel 1</u>

Zu einer Lösung von 4,4 g α-(2,4-Dichlorbenzyl)-α-(2-isopropyl-m-dithianyl-2) -3-pyridinmethanol in 60 ml 4N Schwefelsäure werden 180 mg Quecksilbersulfat gegeben, und das Reaktionsgemisch wird dann 8 Stunden auf Rückflusstemperatur erhitzt. Man lässt das Gemisch auf Raumtemperatur abkühlen, stellt es unter Zusatz von festem Natriumbicarbonat auf pH 8, und verteilt es zwischen Aethylacetat und Wasser. Die organische Phase wird dreimal mit gesättigter wässriger Natriumchloridlösung gewaschen und unter vermindertem Druck eingedampft. Man unterwirft den Rückstand einer Säulenchromatographie an Kieselgel unter Verwendung von Diäthyläther als Laufmittel, kristallisiert ihn aus Methylenchlorid/n-Hexan und erhält dabei 3,0 g 5-(2,4-Dichlorphenyl)-4-hydroxy-2-methyl-4-(3-pyridyl) -3-pentanon, Smp. 144-145°C.

In analoger Weise erhält man ausgehend von:

- α-(2-Aethyl-m-dithianyl-2)-α-(2,4-dichlor -α-methylbenzyl)-3-pyridinmethanol das 1-(2,4-Dichlor-α-methylbenzyl)-1-hydroxy-1-(3-pyridyl) -2-butanon als farblose Kristalle, Smp. 143,5-144°C;

- α-(2-Aethyl-m-dithianyl-2)-α-(2,4-dichlorbenzyl) -3-pyridinmethanol das 5-(2,4-Dichlorphenyl)-4-hydroxy-4-(3-pyridyl) -3-pentanon als farblose Kristalle, Smp. 145-146,5°C;

- α-(2,4-Dichlor-α-methylbenzyl) -α-(m-dithianyl-2)-3-pyridinmethanol den α-(2,4-Dichlor-α-methylbenzyl)-α-hydroxy -3-pyridinacetaldehyd als farbloses Oel;

- α-(2,4-Dichlorbenzyl) -α-(m-dithianyl-2)-3-pyridinmethanol den α-(2,4-Dichlorbenzyl)-α-hydroxy-3-pyridinacetaldehyd als farbloses Oel.

## Beispiel 2

Eine Lösung von 1 g α-(3,3-Diäthoxy-1-propinyl)-α-(2,4-dichlor -α-methylbenzyl)-3-pyridinmethanol in 30 ml 4N Schwefelsäure wird unter Rühren 1,5 Stunden auf 120°C erhitzt, und anschliessend lässt man das Reaktionsgemisch

auf Raumtemperatur abkühlen, neutralisiert es mit festem Natriumbicarbonat und extrahiert es mit Methylenchlorid. Die organische Phase wird dreimal mit gesättigter wässriger Natriumchloridlösung gewaschen und unter vermindertem Druck eingedampft. Danach unterwirft man den Rückstand einer Säulenchromatographie an Kieselgel unter Verwendung von Methylenchlorid/Aethylacetat (4:1) als Laufmittel und kristallisiert ihn aus n-Hexan um. Auf diese Weise erhält man 0,4 g 2-(2,4-Dichlor-α-methylbenzyl)-2-(3-pyridyl) -3(2H)-furanon, Smp. 111-111,5°C.

In analoger Weise erhält man ausgehend von:

- α-(3,3-Diäthoxy-1-propinyl)-α-(2,4-dichlorbenzyl) -3-pyridinmethanol das 2-(2,4-Dichlorbenzyl)-2-(3- -pyridyl)-3(2H)-furanon, Smp. 129-131°C;

- α-Aethinyl-α-(2,4-dichlor-α-methylbenzyl) -3-pyridinmethanol das 4-(2,4-Dichlorphenyl)-3-hydroxy-3-(3-pyridyl)-2-pentanon als farblose Kristalle, Smp. 147°C;

- α-Aethinyl-α-(2,4-dichlorbenzyl)-3-pyridinmethanol das 4-(2,4-Dichlorphenyl)-3-hydroxy-3-(3-pyridyl)-2- -butanon als farblose Kristalle, Smp. 116,5-118,5°C;

- α-Aethinyl-α-(2,4-dichlor -α-propylbenzyl)-3- -pyridinmethanol das 4-(2,4-Dichlorphenyl)-3-hydroxy- -3-(3-pyridyl)-2-heptanon als gelbliche Kristalle, Smp. 133-135°C;

- α-(3,3-Diäthoxy-1-propinyl)-α-(2,4-dichlor -α-propylbenzyl)-3-pyridinmethanol das 2-(2,4-Dichlor-α-propylbenzyl)-2-(3-pyridyl) -3(2H)-furanon als orangefarbene Kristalle, Smp. 112-114°C;

- α-Aethinyl-α-(p-chlorbenzyl)-3-pyridinmethanol das 4-(p-Chlorphenyl)-3-hydroxy-3-(3-pyridyl)-2-butanon als farblose Kristalle, Smp. 114,5-117,5°C;

- α-Aethinyl-α-(p-chlor-α-methylbenzyl) -3-pyridinmethanol das 4-(p-Chlorphenyl)-3-hydroxy-3-(3-pyridyl)-2-pentanon als farblose Kristalle, Smp. 104,5--106,5°C;

- α-(p-Chlor-α-methylbenzyl)-α-(3,3-diäthoxy-1-propinyl)-3-pyridinmethanol das 2-(p-Chlor-α-methylbenzyl)-2-(3-pyridyl)-3(2H)-furanon als orangefarbene Kristalle, Smp. 90-100°C;

- α-Aethinyl-α-(α-äthyl-2,4-dichlorbenzyl)-3-pyridinmethanol das 4-(2,4-Dichlorphenyl)-3-hydroxy-3-(3-pyridyl)-2-hexanon als farblose Kristalle, Smp. 171--174°C.

## Beispiel 3

Zu einer Lösung von 2,2 ml Aethylvinyläther in 10 ml Tetrahydrofuran werden unter Kühlung bei -75°C bis -70°C 10,3 ml einer 1,4-molaren Lösung von tert.Butyllithium in n-Hexan getropft. Anschliessend lässt man das Reaktionsgemisch sich langsam auf -5°C erwärmen, währenddessen die gelbgrüne Suspension sich teilweise entfärbt und klar wird. Man kühlt das Gemisch erneut auf -70°C bis -75°C ab und tropft in diesem Temperaturbereich eine Lösung von 4,0 g 2-(2,4-Dichlorphenyl)-1-(3-pyridyl)-4-pentin-1-on in 14 ml Tetrahydrofuran zu. Nach 20 Minuten bei -75°C lässt man das Reaktionsgemisch sich auf 0°C erwärmen. Zur Aufarbeitung versetzt man das Reaktionsgemisch mit 100 ml gesättigter wässriger Natriumbicarbonatlösung, extrahiert mit Aethylacetat, wäscht die organische Phase dreimal mit gesättigter wässriger Natriumchloridlösung und dampft sie unter vermindertem Druck ein. Anschliessend reinigt man das Rohprodukt

durch Säulenchromatographie an Kieselgel mit Methylenchlorid/Aethylacetat (1:1) und Umkristallisieren aus Methylenchlorid/n-Hexan. Auf diese Weise erhält man 1,0 g α-(1--Aethoxyvinyl)-α-[2,4-dichlor-α -(2-propinyl)-benzyl]-3--pyridinmethanol, Smp. 139°C.

In analoger Weise erhält man ausgehend von:

- 2,4-Dichlorbenzyl-3-pyridyl-keton und Methylvinyläther das α-(2,4-Dichlorbenzyl)-α-(1-methoxyvinyl) -3-pyridinmethanol als farblose Kristalle, Smp. 138-139°C;

- 2-(2,4-Dichlorphenyl)-1-(3-pyridyl)-1-propanon und Methylvinyläther das α-(2,4-Dichlor-α-methylbenzyl)-α-(1-methoxyvinyl) -3-pyridinmethanol als farblose Kristalle, Smp. 140-141°C;

- 2-(2,4-Dichlorphenyl)-1-(3-pyridyl)-4-penten-1-on und Aethylvinyläther das α-(1-Aethoxyvinyl)-α-(α--allyl-2,4-dichlorbenzyl) -3-pyridinmethanol als farblose Kristalle, Smp. 128-129°C;

- 2,4-Dichlorbenzyl-3-pyridyl-keton und Aethylvinyläther das α-(1-Aethoxyvinyl)-α-(2,4-dichlorbenzyl) -3-pyridinmethanol als farblose Kristalle, Smp. 140-141°C;

- 2-(2,4-Dichlorphenyl)-1-(3-pyridyl)-1-propanon und Aethylvinyläther das α-(1-Aethoxyvinyl)-α-(2,4--dichlor-α-methylbenzyl) -3-pyridinmethanol als farblose Kristalle, Smp. 134-136°C;

- 2-(2,4-Dichlorphenyl)-1-(3-pyridyl)-1-propanon und Butylvinyläther das α-(1-Butoxyvinyl)-α-(2,4-dichlor-α-methylbenzyl) -3-pyridinmethanol als farblose Kristalle, Smp. 115-116°C;

- 2-(2,4-Dichlorphenyl)-1-(3-pyridyl)-1-propanon und Isobutylvinyläther das α-(1-Isobutoxyvinyl)-α--(2,4-dichlor -α-methylbenzyl)-3-pyridinmethanol als farblose Kristalle, Smp. 132-133°C;

- 2-(2,4-Dichlorphenyl)-1-(3-pyridyl)-1-propanon und Aethyl-(1-propenyl)-äther das α-(1-Aethoxy-1-propenyl)-α-(2,4-dichlor -α-methylbenzyl)-3-pyridinmethanol als farblose Kristalle, Smp. 120-121°C;

- 2,4-Dichlorbenzyl-3-pyridyl-keton und Butylvinyläther das α-(1-Butoxyvinyl)-α-(2,4-dichlorbenzyl) -3-pyridinmethanol als farblose Kristalle, Smp. 97°C;

- 2,4-Dichlorbenzyl-3-pyridyl-keton und Isobutylvinyläther das α-(1-Isobutoxyvinyl)-α-(2,4-dichlorbenzyl) -3-pyridinmethanol als farblose Kristalle, Smp. 107-108°C;

- 2-(2,4-Dichlorphenyl)-1-(3-pyridyl)-1-pentanon und Aethylvinyläther das α-(1-Aethoxyvinyl)-α-(2,4--dichlor-α-propylbenzyl) -3-pyridinmethanol als farblose Kristalle, Smp. 133-136°C.

## Beispiel 4

Ein Gemisch von 2,0 g α-(1-Aethoxyvinyl)-α-[2,4--dichlor-α-(2-propinyl)-benzyl] -3-pyridinmethanol (siehe Beispiel 3, 1. Endprodukt), 1 ml konzentrierter Salzsäure und 15 ml Methanol als klare gelbe Lösung wird 1 Stunde bei Raumtemperatur gehalten. Danach wird das Gemisch zur Entfernung von Methanol unter vermindertem Druck eingedampft

und der Rückstand mit gesättigter wässriger Natriumbicarbonatlösung neutralisiert. Man extrahiert das wässrige Gemisch mit Aethylacetat, wäscht die organische Phase mit gesättigter wässriger Natriumchloridlösung und dampft ein. Schliesslich wird der Rückstand aus Methylenchlorid/n-Hexan umkristallisiert. Man erhält 1,8 4-(2,4-Dichlorphenyl)-3-hydroxy-3-(3-pyridyl) -6-heptin-2-on, Smp. 132°C.

In analoger Weise erhält man ausgehend von:

- α-(1-Aethoxyvinyl)-α-(α-allyl-2,4-dichlorbenzyl) -3-pyridinmethanol (siehe Beispiel 3, 4. Endprodukt) das 4-(2,4-Dichlorphenyl)-3-hydroxy-3-(3-pyridyl) -6-hepten-2-on als farblose Kristalle, Smp. 124-125°C;

## Beispiel 5

1,2 g 3-(2,4-Dichlorphenyl)-2-(3-pyridyl)-1,2-butandiol werden unter Argon in 19 ml N,N-Dimethylformamid gelöst und bei -15°C mit 0,12 g Natriumhydrid (99%) versetzt. Man lässt das Gemisch 0°C annehmen, rührt es 6 Stunden bei dieser Temperatur und gibt 0,4 ml Aethyljodid zu. Dabei entsteht eine rotorangefarbene Suspension, die man nach 1-stündigem Rühren bei 0°C mit 20 ml gesättigter wässriger Natriumbicarbonatlösung versetzt und anschliessend zur Entfernung des Lösungsmittels unter einem Druck von 0,1 mmHg eindampft. Der Rückstand wird in Wasser aufgenommen, die wässrige Lösung mit Aethylacetat extrahiert, und die organische Phase mit gesättigter wässriger Natriumchloridlösung gewaschen und dann unter vermindertem Druck zur Trockene eingedampft. Man reinigt den öligen Rückstand chromatographisch an Kieselgel mit Diäthyläther/n-Hexan (17:3) und kristallisiert ihn aus Methylenchlorid/n-Hexan um. Auf diese Weise erhält man 0,7 g α-Aethoxymethyl-α-(2,4-dichlor-α-methylbenzyl)-3-pyridinmethanol als farblose Kristalle, Smp. 86°C.

In analoger Weise erhält man ausgehend von:

- 3-(2,4-Dichlorphenyl)-2-(3-pyridyl)-1,2-butandiol und Methyljodid das α-(2,4-Dichlor-α-methylbenzyl)-α-methoxymethyl -3-pyridinmethanol als farblose Kristalle, Smp. 105-107°C;

- 4-(2,4-Dichlorphenyl)-3-(3-pyridyl)-2,3-butandiol und Methyljodid das α-(2,4-Dichlorbenzyl)-α-(1-methoxyäthyl) -3-pyridinmethanol als farblose Kristalle, Smp. 94-95°C;

- 4-(2,4-Dichlorphenyl)-3-(3-pyridyl)-2,3-hexandiol und Methyljodid das α-(α-Aethyl-2,4-dichlorbenzyl)-α-(1-methoxyäthyl) -3-pyridinmethanol als farblose Kristalle, Smp. 155°C.

## Beispiel 6

Ein Gemisch von 2,0 g α-(2,4-Dichlorbenzyl)-α-(1-methoxyvinyl) -3-pyridinmethanol (siehe Beispiel 3, 2. Endprodukt), 0,2 g Hydrierkatalysator (5% Palladium auf Kohle) und 40 ml Essigsäure wird 7 Stunden bei Raumtemperatur unter einer Wasserstoffatmosphäre bei Normaldruck gerührt. Danach wird der Hydrierkatalysator durch Filtration entfernt, das Filtrat unter vermindertem Druck vom Lösungsmittel befreit, der Rückstand in eine gesättigte Natriumbicarbonatlösung aufgenommen und die Lösung mit Aethylacetat extrahiert. Man dampft die organische Phase ein und kristallisiert den Rückstand aus Methylenchlorid/n-Hexan. Auf diese Weise erhält man das α-(2,4-Dichlorbenzyl)-α-(1-methoxyäthyl)-3-pyridinmethanol als farblose Kristalle, Smp. 94-95°C.

## Beispiel 7

Zu einer Lösung von 2,86 g frisch sublimiertem Kalium-tert.butanolat in 145 ml tert.Butyl-methyl-äther

tropft man bei -75°C 18,2 ml einer 1,4-molaren Lösung von sek.Butyllithium in Cyclohexan. Das Gemisch wird 2 Stunden bei dieser Temperatur gerührt und anschliessend mit 25 ml einer 2-molaren Lösung von Lithiumbromid in Tetrahydrofuran versetzt. Die Temperatur des Gemisches wird auf -10°C erhöht und nach 30 Minuten wieder auf -75°C gesenkt. Danach tropft man eine Lösung von 4,84 g 2,4-Dichlorbenzyl-3-pyridylketon in 28 ml Tetrahydrofuran bei -75°C zu, erhöht die Reaktionstemperatur nach 15 Minuten auf 0°C und beendet die Reaktion durch vorsichtige Zugabe von 20 ml gesättigter wässriger Natriumbicarbonatlösung. Das Gemisch wird mit 200 ml Wasser verdünnt und mit Aethylacetat extrahiert. Auf diese Weise erhält man ein Edukt-Produkt-Gemisch (ca. 2:1), das an Kieselgel mit Diäthyläther chromatographisch getrennt wird, und zwar unter anderem in das α-(tert.Butoxymethyl)-α--(2,4-dichlorbenzyl) -3-pyridinmethanol als farbloses Oel.

In analoger Weise erhält man ausgehend von:

- 2,4-Dichlor-α-methylbenzyl-3-pyridylketon und tert.Butyl-methyl-äther das α-(tert.Butoxymethyl)--α-(2,4-dichlor-α-methylbenzyl)-3-pyridinmethanol als farblose Kristalle, Smp. 123-125°C.

## Beispiel 8

Eine Lösung von 1,5 g 1-(2,4-Dichlor-α-methylbenzyl)-1-hydroxy-1-(3-pyridyl) -2-butanon (siehe Beispiel 1, 2. Endprodukt) und 1,4 g O-Methylhydroxylamin-Hydrochlorid in 10 ml Pyridin wird 16 Stunden auf Rückflusstemperatur erhitzt. Anschliessend wird die Lösung zwischen Aethylacetat und Wasser verteilt und die wässrige Phase dreimal mit frischen Mengen Aethylacetat extrahiert. Man wäscht die vereinigten organischen Phasen mit gesättigter wässriger Natriumchloridlösung, dampft das Lösungsmittel unter vermindertem Druck ab und reinigt den Rückstand durch Säulenchromatographie an Kieselgel mit Methylenchlorid/Aethylacetat

(4:1) und Umkristallisieren aus Methylenchlorid/n-Hexan. Dies ergibt 1,3 g farblose Kristalle von 5-(2,4-Dichlorphenyl)-4-hydroxy-4-(3-pyridyl) -3-hexanon-O-methyloxim, Smp. 168-170°C.

In analoger Weise erhält man ausgehend von:

- α-(2,4-Dichlorbenzyl)-α-hydroxy -3-pyridinacetaldehyd (siehe Beispiel 1, 5. Endprodukt) und O-Methylhydroxylamin-Hydrochlorid das α-(2,4-Dichlorbenzyl)--3-pyridinglykolaldehyd-(Z) -O-methyloxim als farblose Kristalle, Smp. 134-135°C;

- α-(2,4-Dichlor-α-methylbenzyl)-α-hydroxy -3-pyridinacetaldehyd (siehe Beispiel 1, 4. Endprodukt) und O-Methylhydroxylamin-Hydrochlorid das α-(2,4-Dichlor-α-methylbenzyl)-α -[(E)-(methoxyimino)-methyl]-3-pyridinmethanol als farblose Kristalle, Smp. 147-148°C;

- 4-(2,4-Dichlorbenzyl)-3-hydroxy-3-(3-pyridyl)-2-butanon (siehe Beispiel 2, 4. Endprodukt) und Hydroxylamin--Hydrochlorid das 4-(2,4-Dichlorbenzyl)-3-hydroxy-3 -(3-pyridyl)-2-butanon-oxim als farblose Kristalle, Smp. 174-178°C;

- 4-(2,4-Dichlorbenzyl)-3-hydroxy-3-(3-pyridyl)-2-butanon (siehe Beispiel 2, 4. Endprodukt) und O-Methylhydroxylamin-Hydrochlorid das 4-(2,4-Dichlorbenzyl)-3--hydroxy-3-(3-pyridyl) -2-butanon-O-methyloxim als farblose Kristalle, Smp. 136-138°C;

- 4-(2,4-Dichlorphenyl)-3-hydroxy-3-(3-pyridyl)-2-pentanon (siehe Beispiel 2, 3. Endprodukt) und Hydroxylamin--Hydrochlorid das 4-(2,4-Dichlorphenyl)-3-hydroxy-3-

-(3-pyridyl) -2-pentanon-oxim als farblose Kristalle, Smp. 174-175°C;

- 4-(2,4-Dichlorphenyl)-3-hydroxy-3-(3-pyridyl)-2-pentanon (siehe Beispiel 2, 3. Endprodukt) und O-Methylhydroxylamin-Hydrochlorid das 4-(2,4-Dichlorphenyl)-3--hydroxy-3-(3-pyridyl) -2-pentanon-O-methyloxim als farblose Kristalle, Smp. 154-157°C;

- α-(2,4-Dichlor-α-methylbenzyl) -α-hydroxy-3--pyridinacetaldehyd (siehe Beispiel 1, 4. Endprodukt) und Hydroxylamin-Hydrochlorid das α-(2,4-Dichlor--α-methylbenzyl) -3-pyridinglykolaldehyd-oxim als farblose Kristalle, Smp. 185-186°C;

- 4-(2,4-Dichlorphenyl)-3-hydroxy-3-(3-pyridyl)-2--hexanon (siehe Beispiel 2, 10. Endprodukt) und Hydroxylamin-Hydrochlorid das 4-(2,4-Dichlorphenyl)-3-hydroxy-3-(3-pyridyl)-2-hexanon-oxim als farblose Kristalle, Smp. 196-199°C.

## Beispiel 9

Zu einer Lösung von 5 g 5-(2,4-Dichlorphenyl)-4-hydroxy-4-(3-pyridyl)-3-pentanon (siehe Beispiel 1, 3. Endprodukt) in 90 ml Toluol werden 27 ml Aethylenglykol und 0,8 ml konzentrierte Schwefelsäure gegeben, und das Reaktionsgemisch wird 24 Stunden an einem Wasserabscheider auf Rückflusstemperatur erhitzt. Nach Erkalten auf Raumtemperatur wird das Gemisch mit gesättigter wässriger Natriumbicarbonatlösung neutralisiert und mit Aethylacetat extrahiert. Man wäscht die organische Phase mit gesättigter wässriger Natriumchloridlösung, dampft das Lösungsmittel unter vermindertem Druck ab und kristallisiert den Rückstand aus Methylenchlorid/n-Hexan um. Auf diese Weise erhält man 5,3 g 5-(2,4-Dichlorphenyl)-4-hydroxy-4-(3-pyridyl) -2-pentanon-

-äthylenketal als farblose Kristalle, Smp. 136-137°C.

In analoger Weise erhält man ausgehend von:

- α-(2,4-Dichlorbenzyl)-α-hydroxy -3-pyridinacetaldehyd (siehe Beispiel 1, 5. Endprodukt) und Aethylenglykol das α-(2,3-Dichlorbenzyl)-α-(1,3-dioxolan--2-yl) -3-pyridinmethanol als farblose Kristalle, Smp. 120-121°C;

- 4-(2,4-Dichlorphenyl)-3-hydroxy-3-(3-pyridyl)-2-butanon (siehe Beispiel 2, 4. Endprodukt) und Aethylenglykol das 4-(2,4-Dichlorphenyl)-3-hydroxy-3-(3-pyridyl) -2-butanon-äthylenketal als farblose Kristalle, Smp. 126-127°C;

- 4-(2,4-Dichlorphenyl)-3-hydroxy-3-(3-pyridyl)-2-pentanon (siehe Beispiel 2, 3. Endprodukt) und Aethylenglykol das 4-(2,4-Dichlorphenyl)-3-hydroxy-3-(3-pyridyl) -2-pentanon-äthylenketal als farblose Kristalle, Smp. 142-144°C;

- 4-(2,4-Dichlorphenyl)-3-hydroxy-3-(3-pyridyl)-2-butanon (siehe Beispiel 2, 4. Endprodukt) und Propan-1,3-diol das 4-(2,4-Dichlorphenyl)-3-hydroxy-3-(3-pyridyl)-2--butanon-trimethylenketal als farblose Kristalle, Smp. 161°C.

## Beispiel 10

Eine Lösung von 4,9 g 4-(2,4-Dichlorphenyl)-3-(3-pyridyl)-2,3-pentandiol in 400 ml Methylenchlorid und 100 ml Tetrahydrofuran wird mit 5,3 g O-Amino-2,4,6-trimethylbenzolsulfonat versetzt, und das Reaktionsgemisch wird 18 Stunden bei Raumtemperatur gerührt. Danach wird das Lösungsmittel unter vermindertem Druck abgedampft und der Rückstand an Kieselgel mit Methylenchlorid/Aethanol (3:2)

chromatographisch gereinigt. Man erhält 3,1 g 1-Amino-3--[2,4-dichlor-α-hydroxy-α-(1-hydroxyäthyl)-ß-methyl-phenäthyl]-pyridinium-2,4,6-trimethylbenzolsulfonat als farbloses Oel.

In analoger Weise erhält man ausgehend von:

- 4-(2,4-Dichlorbenzyl)-3-hydroxy-3-(3-pyridyl)-2-butanon (siehe Beispiel 2, 4. Endprodukt) das 3-(α-Acetyl--2,4-dichlor-α-hydroxyphenäthyl)-1-aminopyridinium--2,4,6-trimethylbenzolsulfonat als farbloses Oel.

II. <u>Herstellung der Ausgangsmaterialien der Formeln II und VI:</u>

## Beispiel 11

Zu einer Lösung von 9,2 g 2-Isopropyl-1,3-dithian in 150 ml Tetrahydrofuran werden unter Kühlung bei -75°C bis -70°C 38,8 ml einer 1,6-molaren Lösung von n-Butyllithium in n-Hexan getropft. Anschliessend lässt man die gelbe Lösung sich langsam auf 0°C erwärmen, rührt sie 10 Minuten bei dieser Temperatur und kühlt sie wieder auf -70°C bis -75°C. Dann tropft man innerhalb dieses Temperaturbereichs während 45 Minuten eine Lösung von 15 g 2,4-Dichlorbenzyl-(3-pyridyl)-keton in 40 ml Tetrahydrofuran zu und lässt das Reaktionsgemisch sich langsam auf Raumtemperatur erwärmen. Man beendigt die Reaktion durch Zugabe von 40 ml Wasser, dampft das Lösungsmittel unter vermindertem Druck ab und nimmt den Rückstand in Diäthyläther auf. Dann wird die ätherische Lösung dreimal mit gesättigter wässriger Natriumchloridlösung gewaschen, die organische Phase unter vermindertem Druck eingedampft und der Rückstand an Kieselgel mit Diäthyläther/n-Hexan (1:1) chromatographisch gereinigt. Auf diese Weise erhält man 8,7 g kristallinen α-(2,4-Dichlor-

benzyl)-α-(2-isopropyl-m-dithianyl-2) -3-pyridinmethanol, Smp. 124-126°C.

In analoger Weise erhält man ausgehend von:

- 2-Aethyl-1,3-dithian, n-Butyllithium und 2-(2,4-Dichlorphenyl)-1-(3-pyridyl)-1-propanon das α-(2--Aethyl-m-dithianyl-2)-α-(2,4-dichlor-α -methylbenzyl)-3-pyridinmethanol als farblose Kristalle, Smp. 100-101°C;

- 2-Aethyl-1,3-dithian, n-Butyllithium und 2,4-Dichlorbenzyl-(3-pyridyl)-keton das α-(2-Aethyl-m-dithianyl-2)-α-(2,4-dichlorbenzyl) -3-pyridinmethanol als farblose Kristalle, Smp. 138-139°C;

- 1,3-Dithian, n-Butyllithium und 2-(2,4-Dichlorphenyl)--1-(3-pyridyl)-1-propanon das α-(2,4-Dichlor-α--methylbenzyl)-α-(m-dithianyl -2)-3-pyridinmethanol als farblose Kristalle, Smp. 124-127°C;

- 1,3-Dithian, n-Butyllithium und 2,4-Dichlorbenzyl-3--(3-pyridyl)-keton das α-(2,4-Dichlorbenzyl)-α-(m--dithianyl-2) -3-pyridinmethanol als gelbe Kristalle, Smp. 142-144°C.

Beispiel 12

Eine Lösung von 5,8 g 4-(2,4-Dichlorphenyl)-3-hydroxy--3-(3-pyridyl)-6-heptin-2-on (siehe Beispiel 4, 1. Endprodukt) in 100 ml Aethanol wird bei Raumtemperatur mit 0,8 g Natriumborhydrid versetzt und das Ganze 30 Minuten gerührt. Danach zersetzt man das überschüssige Natriumborhydrid, indem man die gelbliche Reaktionslösung mit 2N Schwefelsäure auf pH 1 stellt und mit gesättigter wässriger Natriumbicarbonatlösung wieder neutralisiert. Das Gemisch wird zur Entfernung des Lösungsmittels unter vermindertem Druck eingedampft, der Rückstand zwischen Aethylacetat und Wasser verteilt und die organische Phase mit gesättigter wässriger Natriumchloridlösung gewaschen und zur Entfernung des Lösungsmittels unter vermindertem Druck eingedampft. Der Rückstand wird an Kieselgel mit Methylenchlorid/Aethylacetat (3:7) chromatographisch gereinigt. Man erhält auf diese Weise 5,7 g 4-(2,4-Dichlorphenyl)-3-(3-pyridyl)-6-heptin-2,3-diol als farblosen festen Schaum.

In analoger Weise erhält man ausgehend von:

- 4-(2,4-Dichlorphenyl)-3-hydroxy -3-(3-pyridyl)-2-pentanon (siehe Beispiel 2, 3. Endprodukt) das 4-(2,4-Dichlorphenyl)-3-(3-pyridyl)-2,3-pentandiol als farblose Kristalle, Smp. 90-110°C;

- 4-(2,4-Dichlorphenyl)-3-hydroxy-3-(3-pyridyl)-2-butanon (siehe Beispiel 2, 4. Endprodukt) das 4-(2,4-Dichlorphenyl)-3-(3-pyridyl)-2,3-butandiol als farblose Kristalle, Smp. 165-167°C;

- 4-(2,4-Dichlorphenyl)-3-hydroxy-3-(3-pyridyl)-2-heptanon (siehe Beispiel 2, 5. Endprodukt) das 4-(2,4-Dichlorphenyl)-3-(3-pyridyl)-2,3-heptandiol als farblose Kristalle, Smp. 202-204°C;

- α-(2,4-Dichlorbenzyl)-α-hydroxy -3-pyridinacetaldehyd (siehe Beispiel 1, 5. Endprodukt) das 1-(2,4-Dichlorbenzyl)-1-(3-pyridyl)-1,2-äthandiol als farblose Kristalle, Smp. 158-159°C;

- 1-(2,4-Dichlor-α-methylbenzyl)-1-hydroxy -1-(3-pyridyl)-2-butanon (siehe Beispiel 1, 2. Endprodukt) das 5-(2,4-Dichlorphenyl)-4-(3-pyridyl) -3,4-hexandiol als farblosen festen Schaum;

- 4-(2,4-Dichlorphenyl)-3-hydroxy-3-(3-pyridyl) -6-hepten-2-on (siehe Beispiel 4, 2. Endprodukt) das 4-(2,4-Dichlorphenyl)-3-(3-pyridyl)-6-hepten-2,3-diol als farblose Kristalle, Smp. 190°C;

- α-(2,4-Dichlor-α-methylbenzyl)-α-hydroxy-3-pyridinacetaldehyd (siehe Beispiel 1, 4. Endprodukt) das 3-(2,4-Dichlorphenyl)-2-(3-pyridyl)-1,2-butandiol als farblose Kristalle, Smp. 131-132°C;

- 4-(2,4-Dichlorphenyl)-3-hydroxy-3-(3-pyridyl)-2-hexanon (siehe Beispiel 2, 10. Endprodukt) das 4-(2,4-Dichlorphenyl)-3-(3-pyridyl)-2,3-hexandiol als farblose Kristalle, Smp. 172-175°C.

III. Formulierungsbeispiele:

## Beispiel 13

Ein emulgierbares Konzentrat hat folgende Zusammensetzung:

| | | g/Liter |
|---|---|---|
| Wirkstoff der Formel I | | 250 |
| Polyarylphenol-(18)äthoxylat (Emulgator) | | 300 |
| N-Methyl-2-pyrrolidon (Lösungsmittel) | ad | 1 l |

Der Wirkstoff und der Emulgator werden in das Lösungsmittel aufgenommen, wobei eine klare, kältebeständige Lösung entsteht. In Wasser gegossen ergibt sich eine praktisch klare Emulsion, die als Spritzbrühe dient.

Patentansprüche

1. Verbindungen der allgemeinen Formel

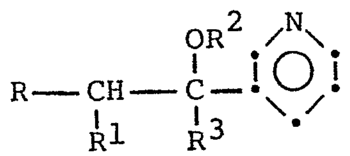

I

worin

R mono-, di- oder trisubstituiertes Phenyl, wobei die Substituenten 1-3 Halogenatome und/oder 1 oder 2 $C_{1-3}$-Alkylgruppen und/oder 1 oder 2 $C_{1-3}$-Alkoxygruppen und/oder 1 oder 2 Trifluormethylgruppen sind,

$R^1$ Wasserstoff, $C_{1-6}$-Alkyl, $C_{2-6}$-Alkenyl oder $C_{2-6}$-Alkinyl,

$R^2$ Wasserstoff,

$R^3$ eine der Gruppen (a) bis (e)

$-CO-R^4$ (a), $-C(OR^5)=CHR^6$ (b),

$-CH(R^4)OR^5$ (c), $-C(R^4)=NOR^7$ (d),

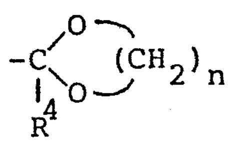

(e)

$R^4$ Wasserstoff oder $C_{1-5}$-Alkyl,

$R^5$ $C_{1-4}$-Alkyl,

$R^6$ und $R^7$ Wasserstoff oder $C_{1-4}$-Alkyl, und

n 2 oder 3 bedeuten, oder

$R^2$ zusammen mit $R^4$ der Gruppe (a), (c), (d) oder (e) Vinylen bedeutet,

sowie die N-Amino-Salze und Säureadditionssalze der Verbindungen der Formel I.

2. Verbindungen nach Anspruch 1, worin R 2,4-Dichlorphenyl bedeutet.

3. Verbindungen nach Anspruch 1 oder 2, worin $R^1$ Wasserstoff oder $C_{1-6}$-Alkyl bedeutet.

4. Verbindungen nach einem der Ansprüche 1 bis 3, worin $R^2$ Wasserstoff bedeutet.

5. Verbindungen nach einem der Ansprüche 1 bis 4, worin $R^3$ Acetyl, 1-Methoxyvinyl oder 1-Aethoxyvinyl bedeutet.

6. Eine Verbindung nach Anspruch 1, ausgewählt aus:

5-(2,4-Dichlorphenyl)-4-hydroxy-4-(3-pyridyl)-3-pentanon,

2-(2,4-Dichlor-α-methylbenzyl)-2-(3-pyridyl)-3(2H)-furanon,

2-(2,4-Dichlorbenzyl)-2-(3-pyridyl)-3(2H)-furanon,

4-(2,4-Dichlorphenyl)-3-hydroxy-3-(3-pyridyl)-2-pentanon,

4-(2,4-Dichlorphenyl)-3-hydroxy-3-(3-pyridyl)-2-butanon,

4-(p-Chlorphenyl)-3-hydroxy-3-(3-pyridyl)-2-pentanon,

α-(2,4-Dichlorbenzyl)-α-(1-methoxyvinyl)-3-pyridinmethanol,

α-Aethoxymethyl-α-(2,4-dichlor-α-methylbenzyl)-3-pyridinmethanol,

α-(2,4-Dichlor-α-methylbenzyl)-α-methoxymethyl-3-pyridinmethanol,

α-(2,4-Dichlorbenzyl)-α-(1-methoxyäthyl)-3-pyridinmethanol,

α-(2,4-Dichlorbenzyl)-3-pyridinglykolaldehyd-O-methyloxim und

α-(2,4-Dichlor-α-methylbenzyl)-α-[(methoxyimino)-methyl]-3-pyridinmethanol.

7. Eine Verbindung nach Anspruch 1, ausgewählt aus:

5-(2,4-Dichlorphenyl)-4-hydroxy-2-methyl-4-(3-pyridyl)-3-pentanon,

1-(2,4-Dichlor-α-methylbenzyl)-1-hydroxy-1-(3-pyridyl)-2-butanon,

α-(2,4-Dichlor-α-methylbenzyl)-α-hydroxy-3-pyridinacetaldehyd,

α-(2,4-Dichlorbenzyl)-α-hydroxy-3-pyridinacetaldehyd,

4-(2,4-Dichlorphenyl)-3-hydroxy-3-(3-pyridyl)-2-heptanon,

2-(2,4-Dichlor-α-propylbenzyl)-2-(3-pyridyl)-3(2H)-furanon,

4-(p-Chlorphenyl)-3-hydroxy-3-(3-pyridyl)-2-butanon,

2-(p-Chlor-α-methylbenzyl)-2-(3-pyridyl)-3(2H)-furanon,

α-(1-Aethoxyvinyl)-α-[2,4-dichlor-α-(2-propinyl)-benzyl]-3-pyridinmethanol,

α-(2,4-Dichlor-α-methylbenzyl)-α-(1-methoxyvinyl)-3-pyridinmethanol,

α-(1-Aethoxyvinyl)-α-(α-allyl-2,4-dichlorbenzyl)-3-pyridinmethanol,

α-(1-Aethoxyvinyl)-α-(2,4-dichlorbenzyl)-3-pyridinmethanol,

α-(1-Aethoxyvinyl)-α-(2,4-dichlor-α-methylbenzyl)-3-pyridinmethanol,

α-(1-Butoxyvinyl)-α-(2,4-dichlor-α-methylbenzyl)-3-pyridinmethanol,

α-(1-Isobutoxyvinyl)-α-(2,4-dichlor-α-methylbenzyl)-3-pyridinmethanol,

α-(1-Aethoxy-1-propenyl)-α-(2,4-dichlor-α-methylbenzyl)-3-pyridinmethanol,

α-(1-Butoxyvinyl)-α-(2,4-dichlorbenzyl)-3-pyridinmethanol,

α-(1-Isobutoxyvinyl)-α-(2,4-dichlorbenzyl)-3-pyridinmethanol,

α-(1-Aethoxyvinyl)-α-(2,4-dichlor-α-propylbenzyl)-3-pyridinmethanol,

4-(2,4-Dichlorphenyl)-3-hydroxy-3-(3-pyridyl)-6-heptin-2-on,

4-(2,4-Dichlorphenyl)-3-hydroxy-3-(3-pyridyl)-6-hepten-2-on,

5-(2,4-Dichlorphenyl)-4-hydroxy-4-(3-pyridyl)-3-hexanon-O-methyloxim,

4-(2,4-Dichlorbenzyl)-3-hydroxy-3-(3-pyridyl)-2-butanon-oxim,

4-(2,4-Dichlorbenzyl)-3-hydroxy-3-(3-pyridyl)-2-butanon-O-methyloxim,

4-(2,4-Dichlorphenyl)-3-hydroxy-3-(3-pyridyl)-2-pentanon-oxim,

4-(2,4-Dichlorphenyl)-3-hydroxy-3-(3-pyridyl)-2-pentanon-O-methyloxim,

α-(2,4-Dichlor-α-methylbenzyl)-3-pyridinglykolal-dehyd-oxim,

5-(2,4-Dichlorphenyl)-4-hydroxy-4-(3-pyridyl)-2-pentanon-äthylenketal,

α-(2,3-Dichlorbenzyl)-α-(1,3-dioxolan-2-yl)-3-pyridinmethanol,

4-(2,4-Dichlorphenyl)-3-hydroxy-3-(3-pyridyl)-2-butanon-äthylenketal,

4-(2,4-Dichlorphenyl)-3-hydroxy-3-(3-pyridyl)-2-pentanon-äthylenketal,

1-Amino-3-[2,4-dichlor-α-hydroxy-α-(1-hydroxy-äthyl)-ß-methylphenäthyl]-pyridinium-2,4,6-trimethylbenzol-sulfonat und

3-(α-Acetyl-2,4-dichlor-α-hydroxyphenäthyl)-1-aminopyridinium-2,4,6-trimethylbenzolsulfonat.

8. Eine Verbindung nach Anspruch 1, ausgewählt aus:

4-(2,4-Dichlorphenyl)-3-hydroxy-3-(3-pyridyl)-2-hexanon,

α-(α-Aethyl-2,4-dichlorbenzyl)-α-(1-methoxyäthyl)-3-pyridinmethanol,

α-(tert.Butoxymethyl)-α-(2,4-dichlorbenzyl)-3-pyridinmethanol,

α-(tert.Butoxymethyl)-α-(2,4-dichlor-α-methylbenzyl)-3-pyridinmethanol,

4-(2,4-Dichlorphenyl)-3-hydroxy-3-(3-pyridyl)-2-hexanon-oxim und

4-(2,4-Dichlorphenyl)-3-hydroxy-3-(3-pyridyl)-2-butanon-trimethylenketal.

9. Verbindungen der allgemeinen Formel

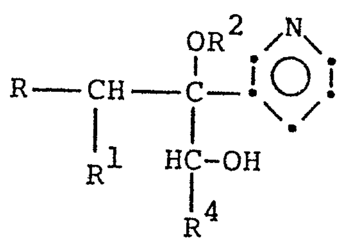

VI

worin

R mono-, di- oder trisubstituiertes Phenyl, wobei die Substituenten 1-3 Halogenatome und/oder 1 oder 2 $C_{1-3}$-Alkylgruppen und/oder 1 oder 2 $C_{1-3}$-Alkoxygruppen und/oder 1 oder 2 Trifluormethylgruppen sind,

$R^1$ Wasserstoff, $C_{1-6}$-Alkyl, $C_{2-6}$-Alkenyl oder $C_{2-6}$-Alkinyl,

$R^2$ Wasserstoff und

$R^4$ Wasserstoff oder $C_{1-5}$-Alkyl bedeuten, oder

$R^2$ zusammen mit $R^4$ Vinylen bedeutet.

10. Eine Verbindung nach Anspruch 9, ausgewählt aus:

4-(2,4-Dichlorphenyl)-3-(3-pyridyl)-2,3-butandiol,

1-(2,4-Dichlorbenzyl)-1-(3-pyridyl)-1,2-äthandiol,

5-(2,4-Dichlorphenyl)-4-(3-pyridyl)-3,4-hexandiol,

3-(2,4-Dichlorphenyl)-2-(3-pyridyl)-1,2-butandiol,

4-(2,4-Dichlorphenyl)-3-(3-pyridyl)-6-heptin-2,3-diol,

4-(2,4-Dichlorphenyl)-3-(3-pyridyl)-2,3-pentandiol und 4-(2,4-Dichlorphenyl)-3-(3-pyridyl)-6-hepten-2,3-diol.

11. 4-(2,4-Dichlorphenyl)-3-(3-pyridyl)-2,3-hexandiol.

12. Fungizides Mittel, dadurch gekennzeichnet, dass es eine wirksame Menge mindestens einer Verbindung der allgemeinen Formel

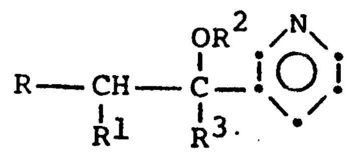

I

worin

R mono-, di- oder trisubstituiertes Phenyl, wobei die Substituenten 1-3 Halogenatome und/oder 1 oder 2 $C_{1-3}$-Alkylgruppen und/oder 1 oder 2 $C_{1-3}$-Alkoxygruppen und/oder 1 oder 2 Trifluormethylgruppen sind,

$R^1$ Wasserstoff, $C_{1-6}$-Alkyl, $C_{2-6}$-Alkenyl oder $C_{2-6}$-Alkinyl,

$R^2$ Wasserstoff,

$R^3$ eine der Gruppen (a) bis (e)

$-CO-R^4$ (a), $-C(OR^5)=CHR^6$ (b),

$-CH(R^4)OR^5$ (c), $-C(R^4)=NOR^7$ (d),

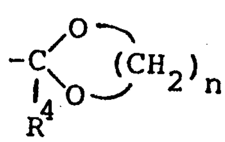

(e)

$R^4$ Wasserstoff oder $C_{1-5}$-Alkyl,

$R^5$ $C_{1-4}$-Alkyl,

$R^6$ und $R^7$ Wasserstoff oder $C_{1-4}$-Alkyl, und

n 2 oder 3 bedeuten, oder

$R^2$ zusammen mit $R^4$ der Gruppe (a), (c), (d) oder (e) Vinylen bedeutet,

oder eines N-Amino-Salzes oder Säureadditionssalzes einer

solchen Verbindung, sowie Formulierungshilfsstoffe enthält.

13. Fungizides Mittel nach Anspruch 12, dadurch gekennzeichnet, dass es eine wirksame Menge mindestens einer aus der Gruppe

5-(2,4-Dichlorphenyl)-4-hydroxy-4-(3-pyridyl)-3-pentanon,
2-(2,4-Dichlor-α-methylbenzyl)-2-(3-pyridyl)-3(2H)-furanon,
2-(2,4-Dichlorbenzyl)-2-(3-pyridyl)-3(2H)-furanon,
4-(2,4-Dichlorphenyl)-3-hydroxy-3-(3-pyridyl)-2-pentanon,
4-(2,4-Dichlorphenyl)-3-hydroxy-3-(3-pyridyl)-2-butanon,
4-(p-Chlorphenyl)-3-hydroxy-3-(3-pyridyl)-2-pentanon,
α-(2,4-Dichlorbenzyl)-α-(1-methoxyvinyl)-3-pyridinmethanol,
α-Aethoxymethyl-α-(2,4-dichlor-α-methylbenzyl)-3-pyridinmethanol,
α-(2,4-Dichlor-α-methylbenzyl)-α-methoxymethyl-3-pyridinmethanol,
α-(2,4-Dichlorbenzyl)-α-(1-methoxyäthyl)-3-pyridinmethanol,
α-(2,4-Dichlorbenzyl)-3-pyridinglykolaldehyd-O-methyloxim und
α-(2,4-Dichlor-α-methylbenzyl)-α-[(methoxyimino)-methyl]-3-pyridinmethanol

ausgewählten Verbindung sowie Formulierungshilfsstoffe enthält.

14. Fungizides Mittel, dadurch gekennzeichnet, dass es eine wirksame Menge mindestens einer aus der Gruppe

4-(2,4-Dichlorphenyl)-3-(3-pyridyl)-2,3-butandiol,
1-(2,4-Dichlorbenzyl)-1-(3-pyridyl)-1,2-äthandiol,
5-(2,4-Dichlorphenyl)-4-(3-pyridyl)-3,4-hexandiol,
3-(2,4-Dichlorphenyl)-2-(3-pyridyl)-1,2-butandiol,

4-(2,4-Dichlorphenyl)-3-(3-pyridyl)-6-heptin-2,3-diol, 4-(2,4-Dichlorphenyl)-3-(3-pyridyl)-2,3-pentandiol und 4-(2,4-Dichlorphenyl)-3-(3-pyridyl)-6-hepten-2,3-diol ausgewählten Verbindung sowie Formulierungshilfsstoffe enthält.

15. Fungizides Mittel, dadurch gekennzeichnet, dass es eine wirksame Menge von 4-(2,4-Dichlorphenyl)-3-(3-pyridyl)-2,3-hexandiol sowie Formulierungshilfsstoffe enthält.

16. Verfahren zur Herstellung von Verbindungen der allgemeinen Formel

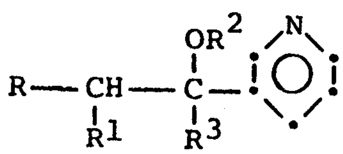

I

worin

R mono-, di- oder trisubstituiertes Phenyl, wobei die Substituenten 1-3 Halogenatome und/oder 1 oder 2 $C_{1-3}$-Alkylgruppen und/oder 1 oder 2 $C_{1-3}$-Alkoxygruppen und/oder 1 oder 2 Trifluormethylgruppen sind,

$R^1$ Wasserstoff, $C_{1-6}$-Alkyl, $C_{2-6}$-Alkenyl oder $C_{2-6}$-Alkinyl,

$R^2$ Wasserstoff,

$R^3$ eine der Gruppen (a) bis (e)

$-CO-R^4$ (a), $-C(OR^5)=CHR^6$ (b),

$-CH(R^4)OR^5$ (c), $-C(R^4)=NOR^7$ (d),

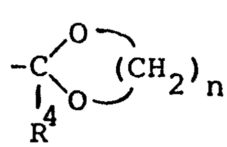

(e)

$R^4$ Wasserstoff oder $C_{1-5}$-Alkyl,

$R^5$ $C_{1-4}$-Alkyl,

$R^6$ und $R^7$ Wasserstoff oder $C_{1-4}$-Alkyl, und

n 2 oder 3 bedeuten, oder
$R^2$ zusammen mit $R^4$ der Gruppe (a), (c), (d) oder (e) Vinylen bedeutet,

und von deren N-Amino-Salzen und Säureadditionssalzen, dadurch gekennzeichnet, dass man

a) zwecks Herstellung derjenigen Verbindungen der Formel I, in denen $R^2$ Wasserstoff, $R^3$ eine Gruppe (a) und $R^4$ Wasserstoff oder $C_{1-5}$-Alkyl bedeuten, ein Dithianderivat der allgemeinen Formel

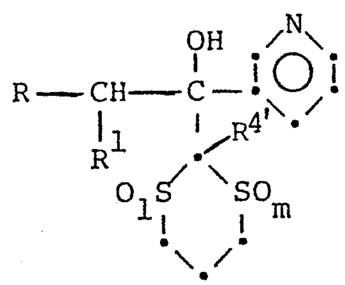

II

worin
R und $R^1$ die oben angegebenen Bedeutung besitzen,
$R^{4'}$ Wasserstoff oder $C_{1-5}$-Alkyl bedeutet und
l und m unabhängig voneinander 0 oder 1 bedeuten,
einer Hydrolyse in saurem Milieu unterwirft,

b) zwecks Herstellung derjenigen Verbindungen der Formel I, in denen $R^3$ eine Gruppe (a) und $R^4$ $C_{1-5}$-Alkyl oder zusammen mit $R^2$ Vinylen bedeuten, ein Alkin der allgemeinen Formel

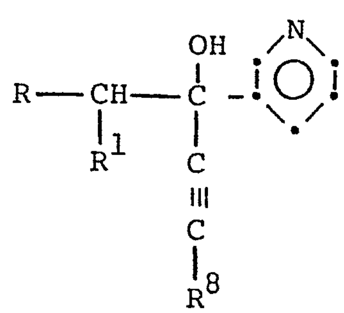

III

worin
R und $R^1$ die oben angegebenen Bedeutungen besitzen,

$R^8$ Wasserstoff, $C_{1-4}$-Alkyl oder eine Gruppe

$$-CH(OR^9)_2$$

bedeutet und
$R^9$ einen niederen Alkylrest bedeutet,
einer Hydrolyse in saurem Milieu unterwirft,

c) zwecks Herstellung derjenigen Verbindungen der Formel I, in denen $R^3$ eine Gruppe (b) bedeutet, ein Keton der allgemeinen Formel

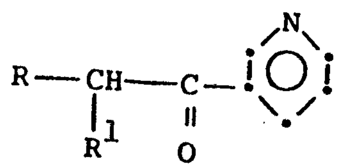
IV

worin R und $R^1$ die oben angegebenen Bedeutungen besitzen,

mit einem geeigneten Metallsalz eines Enoläthers der allgemeinen Formel

$$CH(OR^5)=CHR^6 \qquad V$$

worin $R^5$ und $R^6$ die oben angegebenen Bedeutungen besitzen,

umsetzt,

d) zwecks Herstellung derjenigen Verbindungen der allgemeinen Formel I, in denen $R^2$ Wasserstoff, $R^3$ eine Gruppe (a) und $R^4$ $C_{1-5}$-Alkyl bedeuten, einen Enoläther der allgemeinen Formel

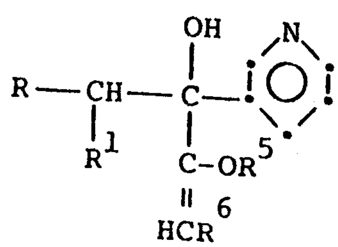
I'

worin

R, $R^1$, $R^5$ und $R^6$ die oben angegebenen Bedeutungen besitzen,

einer Hydrolyse in saurem Milieu unterwirft,

e) zwecks Herstellung derjenigen Verbindungen der Formel I, in denen $R^3$ eine Gruppe (c) bedeutet, einen Alkohol der allgemeinen Formel

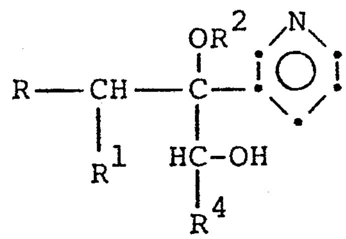

VI

worin

R, $R^1$, $R^2$ und $R^4$ die oben angegebenen Bedeutungen besitzen,

mit einem Alkylierungsmittel behandelt,

f) zwecks Herstellung derjenigen Verbindungen der Formel I, in denen $R^2$ Wasserstoff, $R^3$ eine Gruppe (c) und $R^4$ $C_{1-5}$-Alkyl bedeuten, einen Enoläther der oben angegebenen allgemeinen Formel I' katalytisch hydriert,

g) zwecks Herstellung derjenigen Verbindungen der Formel I, in denen $R^2$ Wasserstoff, $R^3$ eine Gruppe (c) und $R^4$ $C_{1-5}$-Alkyl bedeuten, ein Keton der oben angegebenen allgemeinen Formel IV mit einem geeigneten Metallsalz eines Dialkyläthers der allgemeinen Formel

$$R^{4''}CH_2OR^5 \qquad VII$$

worin

$R^{4''}$ $C_{1-5}$-Alkyl bedeutet und

$R^5$ die oben angegebene Bedeutung besitzt,

umsetzt,

h) zwecks Herstellung derjenigen Verbindungen der Formel I, in denen $R^3$ eine Gruppe (d) bedeutet, einen Aldehyd bzw. ein Keton der allgemeinen Formel

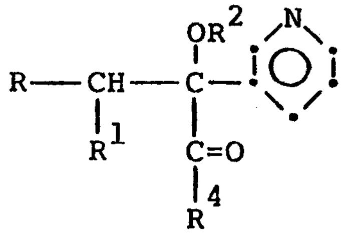

I"

worin
R, $R^1$, $R^2$ und $R^4$ die oben angegebenen Bedeutungen besitzen,
mit einem Amin der allgemeinen Formel

$$H_2N\text{-}OR^7$$ VIII

worin $R^7$ die oben angegebene Bedeutung besitzt,
oder mit einem Salz dieses Amins mit einer starken Säure umsetzt,

i) zwecks Herstellung derjenigen Verbindungen der Formel I, in denen $R^3$ eine Gruppe (e) bedeutet, einen Aldehyd bzw. ein Keton der oben angegebenen allgemeinen Formel I" mit Aethylenglykol bzw. Propandiol-1,3 umsetzt,

und zwecks Herstellung eines N-Amino-Salzes der Verbindung der Formel I diese mit einem Aminderivat der allgemeinen Formel

$$H_2N\text{-}X$$ IX

wobei $X^{\ominus}$ das Anion einer physiologisch verträglichen Säure bedeutet,
N-aminiert

und zwecks Herstellung eines Säureadditionssalzes einer Verbindung der Formel I die Verbindung der Formel I mit einer Säure umsetzt.

17. Verfahren nach Anspruch 16, dadurch gekennzeichnet, dass man eine der Varianten a) bis f), h) und i) und gegebenenfalls die fakultative N-Aminierung bzw. die Ueberführung in ein Säureadditionssalz durchführt.

18. Verfahren zur Bekämpfung von Fungi in der Landwirtschaft und im Gartenbau, dadurch gekennzeichnet, dass man das zu schützende Gut mit einer wirksamen Menge einer Verbindung gemäss einem der Ansprüche 1 bis 7 bzw. eines Mittels gemäss Anspruch 12 oder 13 behandelt.

19. Verfahren zur Bekämpfung von Fungi in der Landwirtschaft und im Gartenbau, dadurch gekennzeichnet, dass man das zu schützende Gut mit einer wirksamen Menge einer Verbindung gemäss Anspruch 8 bzw. eines diese Verbindung enthaltenden Mittels behandelt.

20. Verwendung einer Verbindung gemäss einem der Ansprüche 1 bis 7 bzw. eines Mittels gemäss Anspruch 12 oder 13 zur Bekämpfung von Fungi in der Landwirtschaft und im Gartenbau.

21. Verwendung einer Verbindung gemäss Anspruch 8 bzw. eines diese Verbindung enthaltenden Mittels zur Bekämpfung von Fungi in der Landwirtschaft und im Gartenbau.

***

Patentansprüche für Oesterreich

1. Fungizides Mittel, dadurch gekennzeichnet, dass es eine wirksame Menge mindestens einer Verbindung der allgemeinen Formel

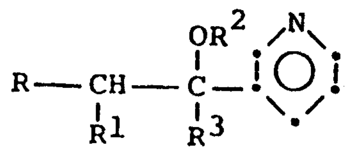

I

worin

R mono-, di- oder trisubstituiertes Phenyl, wobei die Substituenten 1-3 Halogenatome und/oder 1 oder 2 $C_{1-3}$-Alkylgruppen und/oder 1 oder 2 $C_{1-3}$-Alkoxygruppen und/oder 1 oder 2 Trifluormethylgruppen sind,

$R^1$ Wasserstoff, $C_{1-6}$-Alkyl, $C_{2-6}$-Alkenyl oder $C_{2-6}$--Alkinyl,

$R^2$ Wasserstoff,

$R^3$ eine der Gruppen (a) bis (e)

$-CO-R^4$ (a), $-C(OR^5)=CHR^6$ (b),

$-CH(R^4)OR^5$ (c), $-C(R^4)=NOR^7$ (d),

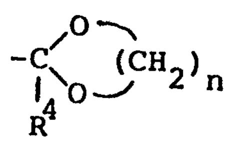

(e)

$R^4$ Wasserstoff oder $C_{1-5}$-Alkyl,

$R^5$ $C_{1-4}$-Alkyl,

$R^6$ und $R^7$ Wasserstoff oder $C_{1-4}$-Alkyl, und

n 2 oder 3 bedeuten, oder

$R^2$ zusammen mit $R^4$ der Gruppe (a), (c), (d) oder (e) Vinylen bedeutet,

oder eines N-Amino-Salzes oder Säureadditionssalzes einer solchen Verbindung, sowie Formulierungshilfsstoffe enthält.

2. Fungizides Mittel nach Anspruch 1, worin R 2,4-Dichlorphenyl bedeutet.

3. Fungizides Mittel nach Anspruch 1 oder 2, worin $R^1$ Wasserstoff oder $C_{1-6}$-Alkyl bedeutet.

4. Fungizides Mittel nach einem der Ansprüche 1 bis 3, worin $R^2$ Wasserstoff bedeutet.

5. Fungizides Mittel nach einem der Ansprüche 1 bis 4, worin $R^3$ Acetyl, 1-Methoxyvinyl oder 1-Aethoxyvinyl bedeutet.

6. Fungizides Mittel nach Anspruch 1, dadurch gekennzeichnet, dass es eine wirksame Menge mindestens einer aus der Gruppe

5-(2,4-Dichlorphenyl)-4-hydroxy-4-(3-pyridyl)-3-pentanon,
2-(2,4-Dichlor-α-methylbenzyl)-2-(3-pyridyl)-3(2H)-furanon,
2-(2,4-Dichlorbenzyl)-2-(3-pyridyl)-3(2H)-furanon,
4-(2,4-Dichlorphenyl)-3-hydroxy-3-(3-pyridyl)-2-pentanon,
4-(2,4-Dichlorphenyl)-3-hydroxy-3-(3-pyridyl)-2-butanon,
4-(p-Chlorphenyl)-3-hydroxy-3-(3-pyridyl)-2-pentanon,
α-(2,4-Dichlorbenzyl)-α-(1-methoxyvinyl)-3-pyridinmethanol,
α-Aethoxymethyl-α-(2,4-dichlor-α-methylbenzyl)--3-pyridinmethanol,
α-(2,4-Dichlor-α-methylbenzyl)-α-methoxymethyl--3-pyridinmethanol,
α-(2,4-Dichlorbenzyl)-α-(1-methoxyäthyl)-3-pyridinmethanol,
α-(2,4-Dichlorbenzyl)-3-pyridinglykolaldehyd-O-methyloxim und

α-(2,4-Dichlor-α-methylbenzyl)-α-[(methoxyimino)-methyl]-3-pyridinmethanol
ausgewählten Verbindung sowie Formulierungshilfsstoffe enthält.

7. Fungzides Mittel nach Anspruch 1, dadurch gekennzeichnet, dass es eine wirksame Menge mindestens einer aus der Gruppe

5-(2,4-Dichlorphenyl)-4-hydroxy-2-methyl-4-(3-pyridyl)-3-pentanon,

1-(2,4-Dichlor-α-methylbenzyl)-1-hydroxy-1-(3-pyridyl)-2-butanon,

α-(2,4-Dichlor-α-methylbenzyl)-α-hydroxy-3-pyridinacetaldehyd,

α-(2,4-Dichlorbenzyl)-α-hydroxy-3-pyridinacetaldehyd,

4-(2,4-Dichlorphenyl)-3-hydroxy-3-(3-pyridyl)-2-heptanon,

2-(2,4-Dichlor-α-propylbenzyl)-2-(3-pyridyl)-3(2H)-furanon,

4-(p-Chlorphenyl)-3-hydroxy-3-(3-pyridyl)-2-butanon,

2-(p-Chlor-α-methylbenzyl)-2-(3-pyridyl)-3(2H)-furanon,

α-(1-Aethoxyvinyl)-α-[2,4-dichlor-α-(2-propinyl)-benzyl]-3-pyridinmethanol,

α-(2,4-Dichlor-α-methylbenzyl)-α-(1-methoxyvinyl)-3-pyridinmethanol,

α-(1-Aethoxyvinyl)-α-(α-allyl-2,4-dichlorbenzyl)-3-pyridinmethanol,

α-(1-Aethoxyvinyl)-α-(2,4-dichlorbenzyl)-3-pyridinmethanol,

α-(1-Aethoxyvinyl)-α-(2,4-dichlor-α-methylbenzyl)-3-pyridinmethanol,

α-(1-Butoxyvinyl)-α-(2,4-dichlor-α-methylbenzyl)-3-pyridinmethanol,

α-(1-Isobutoxyvinyl)-α-(2,4-dichlor-α-methylbenzyl)-3-pyridinmethanol,

α-(1-Aethoxy-1-propenyl)-α-(2,4-dichlor-α-methylbenzyl)-3-pyridinmethanol,

α-(1-Butoxyvinyl)-α-(2,4-dichlorbenzyl)-3-pyridinmethanol,

α-(1-Isobutoxyvinyl)-α-(2,4-dichlorbenzyl)-3-pyridinmethanol,

α-(1-Aethoxyvinyl)-α-(2,4-dichlor-α-propylbenzyl)-3-pyridinmethanol,

4-(2,4-Dichlorphenyl)-3-hydroxy-3-(3-pyridyl)-6-heptin-2-on,

4-(2,4-Dichlorphenyl)-3-hydroxy-3-(3-pyridyl)-6-hepten-2-on,

5-(2,4-Dichlorphenyl)-4-hydroxy-4-(3-pyridyl)-3-hexanon-O-methyloxim,

4-(2,4-Dichlorbenzyl)-3-hydroxy-3-(3-pyridyl)-2-butanonoxim,

4-(2,4-Dichlorbenzyl)-3-hydroxy-3-(3-pyridyl)-2-butanon-O-methyloxim,

4-(2,4-Dichlorphenyl)-3-hydroxy-3-(3-pyridyl)-2-pentanon-oxim,

4-(2,4-Dichlorphenyl)-3-hydroxy-3-(3-pyridyl)-2-pentanon-O-methyloxim,

α-(2,4-Dichlor-α-methylbenzyl)-3-pyridinglykolaldehyd-oxim,

5-(2,4-Dichlorphenyl)-4-hydroxy-4-(3-pyridyl)-2-pentanon-äthylenketal,

α-(2,3-Dichlorbenzyl)-α-(1,3-dioxolan-2-yl)-3-pyridinmethanol,

4-(2,4-Dichlorphenyl)-3-hydroxy-3-(3-pyridyl)-2-butanon-äthylenketal,

4-(2,4-Dichlorphenyl)-3-hydroxy-3-(3-pyridyl)-2-pentanon-äthylenketal,

1-Amino-3-[2,4-dichlor-α-hydroxy-α-(1-hydroxyäthyl)-ß-methylphenäthyl]-pyridinium-2,4,6-trimethylbenzolsulfonat und

3-(α-Acetyl-2,4-dichlor-α-hydroxyphenäthyl)-1-aminopyridinium-2,4,6-trimethylbenzolsulfonat ausgewählten Verbindung sowie Formulierungshilfsstoffe enthält.

8. Fungizides Mittel nach Anspruch 1, dadurch gekennzeichnet, dass es eine wirksame Menge mindestens einer aus der Gruppe

4-(2,4-Dichlorphenyl)-3-hydroxy-3-(3-pyridyl)-2-hexanon,
α-(α-Aethyl-2,4-dichlorbenzyl)-α-(1-methoxyäthyl)-3-pyridinmethanol,
α-(tert.Butoxymethyl)-α-(2,4-dichlorbenzyl)-3-pyridinmethanol,
α-(tert.Butoxymethyl)-α-(2,4-dichlor-α-methylbenzyl)-3-pyridinmethanol,
4-(2,4-Dichlorphenyl)-3-hydroxy-3-(3-pyridyl)-2-hexanon-oxim und
4-(2,4-Dichlorphenyl)-3-hydroxy-3-(3-pyridyl)-2-butanon-trimethylenketal

ausgewählten Verbindung sowie Formulierungshilfsstoffe enthält.

9. Fungizides Mittel, dadurch gekennzeichnet, dass es eine wirksame Menge mindestens einer aus der Gruppe

4-(2,4-Dichlorphenyl)-3-(3-pyridyl)-2,3-butandiol,
1-(2,4-Dichlorbenzyl)-1-(3-pyridyl)-1,2-äthandiol,
5-(2,4-Dichlorphenyl)-4-(3-pyridyl)-3,4-hexandiol,
3-(2,4-Dichlorphenyl)-2-(3-pyridyl)-1,2-butandiol,
4-(2,4-Dichlorphenyl)-3-(3-pyridyl)-6-heptin-2,3-diol,
4-(2,4-Dichlorphenyl)-3-(3-pyridyl)-2,3-pentandiol und
4-(2,4-Dichlorphenyl)-3-(3-pyridyl)-6-hepten-2,3-diol

ausgewälten Verbindung sowie Formulierungshilfsstoffe enthält.

10. Fungizides Mittel, dadurch gekennzeichnet, dass es eine wirksame Menge von 4-(2,4-Dichlorphenyl)-3-(3-pyridyl)-2,3-hexandiol sowie Formulierungshilfsstoffe enthält.

11. Verfahren zur Herstellung von Verbindungen der allgemeinen Formel

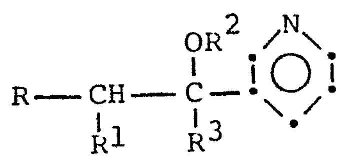

I

worin

R mono-, di- oder trisubstituiertes Phenyl, wobei die Substituenten 1-3 Halogenatome und/oder 1 oder 2 $C_{1-3}$-Alkylgruppen und/oder 1 oder 2 $C_{1-3}$-Alkoxygruppen und/oder 1 oder 2 Trifluormethylgruppen sind,

$R^1$ Wasserstoff, $C_{1-6}$-Alkyl, $C_{2-6}$-Alkenyl oder $C_{2-6}$-Alkinyl,

$R^2$ Wasserstoff,

$R^3$ eine der Gruppen (a) bis (e)

$-CO-R^4$ (a), $-C(OR^5)=CHR^6$ (b),

$-CH(R^4)OR^5$ (c), $-C(R^4)=NOR^7$ (d),

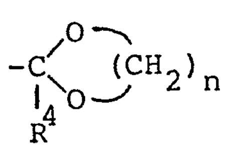

(e)

$R^4$ Wasserstoff oder $C_{1-5}$-Alkyl,

$R^5$ $C_{1-4}$-Alkyl,

$R^6$ und $R^7$ Wasserstoff oder $C_{1-4}$-Alkyl, und

n 2 oder 3 bedeuten, oder

$R^2$ zusammen mit $R^4$ der Gruppe (a), (c), (d) oder (e) Vinylen bedeutet,

und von deren N-Amino-Salzen und Säureadditionssalzen, dadurch gekennzeichnet, dass man

a) zwecks Herstellung derjenigen Verbindungen der Formel I, in denen $R^2$ Wasserstoff, $R^3$ eine Gruppe (a) und $R^4$ Wasserstoff oder $C_{1-5}$-Alkyl bedeuten, ein Dithianderivat der allgemeinen Formel

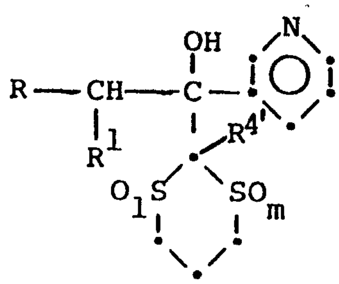

II

worin
R und $R^1$ die oben angegebenen Bedeutung besitzen,
$R^{4'}$ Wasserstoff oder $C_{1-5}$-Alkyl bedeutet und
l und m unabhängig voneinander 0 oder 1 bedeuten,
einer Hydrolyse in saurem Milieu unterwirft,

b) zwecks Herstellung derjenigen Verbindungen der Formel I, in denen $R^3$ eine Gruppe (a) und $R^4$ $C_{1-5}$-Alkyl oder zusammen mit $R^2$ Vinylen bedeuten, ein Alkin der allgemeinen Formel

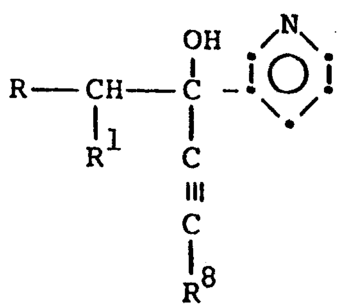

III

worin
R und $R^1$ die oben angegebenen Bedeutungen besitzen,
$R^8$ Wasserstoff, $C_{1-4}$-Alkyl oder eine Gruppe

$$-CH(OR^9)_2$$

bedeutet und
$R^9$ einen niederen Alkylrest bedeutet,

einer Hydrolyse in saurem Milieu unterwirft,

c) zwecks Herstellung derjenigen Verbindungen der Formel I, in denen $R^3$ eine Gruppe (b) bedeutet, ein Keton der allgemeinen Formel

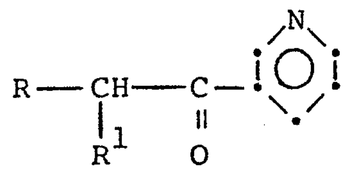

IV

worin R und $R^1$ die oben angegebenen Bedeutungen besitzen,

mit einem geeigneten Metallsalz eines Enoläthers der allgemeinen Formel

$$CH(OR^5)=CHR^6 \qquad V$$

worin $R^5$ und $R^6$ die oben angegebenen Bedeutungen besitzen,

umsetzt,

d) zwecks Herstellung derjenigen Verbindungen der allgemeinen Formel I, in denen $R^2$ Wasserstoff, $R^3$ eine Gruppe (a) und $R^4$ $C_{1-5}$-Alkyl bedeuten, einen Enoläther der allgemeinen Formel

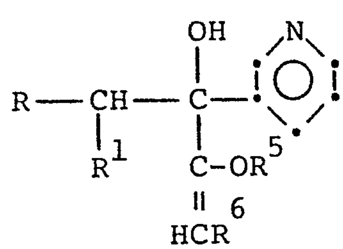

I'

worin

R, $R^1$, $R^5$ und $R^6$ die oben angegebenen Bedeutungen besitzen,

einer Hydrolyse in saurem Milieu unterwirft,

e) zwecks Herstellung derjenigen Verbindungen der Formel I, in denen $R^3$ eine Gruppe (c) bedeutet, einen Alkohol der allgemeinen Formel

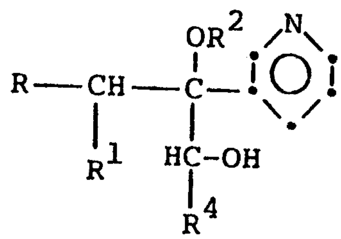
VI

worin
$R$, $R^1$, $R^2$ und $R^4$ die oben angegebenen Bedeutungen besitzen,
mit einem Alkylierungsmittel behandelt,

f) zwecks Herstellung derjenigen Verbindungen der Formel I, in denen $R^2$ Wasserstoff, $R^3$ eine Gruppe (c) und $R^4$ $C_{1-5}$-Alkyl bedeuten, einen Enoläther der oben angegebenen allgemeinen Formel I' katalytisch hydriert,

g) zwecks Herstellung derjenigen Verbindungen der Formel I, in denen $R^2$ Wasserstoff, $R^3$ eine Gruppe (c) und $R^4$ $C_{1-5}$-Alkyl bedeuten, ein Keton der oben angegebenen allgemeinen Formel IV mit einem geeigneten Metallsalz eines Dialkyläthers der allgemeinen Formel

$$R^{4''}CH_2OR^5 \qquad VII$$

worin
$R^{4''}$ $C_{1-5}$-Alkyl bedeutet und
$R^5$ die oben angegebene Bedeutung besitzt,
umsetzt,

h) zwecks Herstellung derjenigen Verbindungen der Formel I, in denen $R^3$ eine Gruppe (d) bedeutet, einen Aldehyd bzw. ein Keton der allgemeinen Formel

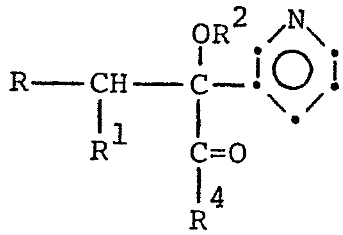

I"

worin
R, $R^1$, $R^2$ und $R^4$ die oben angegebenen Bedeutungen besitzen,
mit einem Amin der allgemeinen Formel

$$H_2N\text{-}OR^7 \quad \text{VIII}$$

worin $R^7$ die oben angegebene Bedeutung besitzt,
oder mit einem Salz dieses Amins mit einer starken Säure umsetzt,

i) zwecks Herstellung derjenigen Verbindungen der Formel I, in denen $R^3$ eine Gruppe (e) bedeutet, einen Aldehyd bzw. ein Keton der oben angegebenen allgemeinen Formel I" mit Aethylenglykol bzw. Propandiol-1,3 umsetzt,

und zwecks Herstellung eines N-Amino-Salzes der Verbindung der Formel I diese mit einem Aminderivat der allgemeinen Formel

$$H_2N\text{-}X \quad \text{IX}$$

wobei $X^{\ominus}$ das Anion einer physiologisch verträglichen Säure bedeutet,
N-aminiert

und zwecks Herstellung eines Säureadditionssalzes einer Verbindung der Formel I die Verbindung der Formel I mit einer Säure umsetzt.

12. Verfahren nach Anspruch 11, dadurch gekennzeichnet, dass man eine der Varianten a) bis f), h) und i) und gegebenenfalls die fakultative N-Aminierung bzw. die Ueberführung in ein Säureadditionssalz durchführt.

13. Verfahren zur Herstellung eines fungiziden Mittels, dadurch gekennzeichnet, dass man mindestens eine der in Anspruch 1 genannten Verbindungen mit Formulierungshilfsstoffen vermischt.

14. Verfahren zur Bekämpfung von Fungi in der Landwirtschaft und im Gartenbau, dadurch gekennzeichnet, dass man das zu schützende Gut mit einer wirksamen Menge eines Mittels gemäss einem der Ansprüche 1 bis 7 behandelt.

15. Verfahren zur Bekämpfung von Fungi in der Landwirtschaft und im Gartenbau, dadurch gekennzeichnet, dass man das zu schützende Gut mit einer wirksamen Menge eines Mittels gemäss Anspruch 8 behandelt.

16. Verwendung eines Mittels gemäss einem der Ansprüche 1 bis 7 zur Bekämpfung von Fungi in der Landwirtschaft und im Gartenbau.

17. Verwendung eines Mittels gemäss Anspruch 8 zur Bekämpfung von Fungi in der Landwirtschaft und im Gartenbau.

***